(19) Europäisches Patentamt / European Patent Office / Office européen des brevets

(11) **EP 4 723 126 A1**

(12) **EUROPEAN PATENT APPLICATION**
published in accordance with Art. 153(4) EPC

(43) Date of publication:
**08.04.2026 Bulletin 2026/15**

(21) Application number: **24814421.4**

(22) Date of filing: **28.05.2024**

(51) International Patent Classification (IPC):
***G16H 40/20*** (2018.01)

(52) Cooperative Patent Classification (CPC):
**G16H 40/20**

(86) International application number:
**PCT/CN2024/095713**

(87) International publication number:
**WO 2024/245224 (05.12.2024 Gazette 2024/49)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **01.06.2023 PCT/CN2023/097857**

(71) Applicant: **Shenzhen Mindray Bio-Medical
Electronics Co., Ltd.
Shenzhen, Guangdong 518057 (CN)**

(72) Inventors:
• **ZHENG, Wenbo**
  **Shenzhen, Guangdong 518057 (CN)**
• **CHEN, Pengzhen**
  **Shenzhen, Guangdong 518057 (CN)**
• **YE, Bo**
  **Shenzhen, Guangdong 518057 (CN)**
• **QI, Huan**
  **Shenzhen, Guangdong 518057 (CN)**
• **LIU, Yan**
  **Shenzhen, Guangdong 518057 (CN)**
• **YANG, Cheng**
  **Shenzhen, Guangdong 518057 (CN)**

(74) Representative: **KIPA AB
Drottninggatan 11
252 21 Helsingborg (SE)**

(54) **METHOD AND SYSTEM FOR PERFORMING REAL-TIME QUALITY CONTROL ON BASIS OF PATIENT SAMPLE, AND METHOD FOR CONSTRUCTING QUALITY CONTROL MODEL**

(57) Embodiments of the present application relate to a method and a system for patient-based real-time quality control, and a method for constructing a quality control model for patient-based real-time quality control. The method includes: while a testing instrument performs real-time tests on current patient samples for a target item, acquiring actual test results of the current patient samples for the target item obtained by the testing instrument and patient information of the current patient samples, wherein the patient information comprises at least one of patient's disease, patient's age, patient's gender or ordering department for the target item; quantifying the patient information of the current patient samples and inputting the quantified patient information into a pre-constructed machine learning model to obtain output results of the machine learning model as predicted test results for the target item, wherein the machine learning model is trained by using actual test results for the target item and patient information of a plurality of historical patient samples; acquiring monitoring indicators based on the actual test results and the predicted test results; and determining whether or not the testing instrument is under control, and/or determining whether or not to give an alarm prompt for indicating that the testing instrument is not under control, based on the monitoring indicators. The obtained quality control has high stability and sensitivity.

EP 4 723 126 A1

100

S110

acquiring actual test results for a target item and patient information of patient samples

S120

quantifying the patient information and inputting the quantified patient information into a machine learning model to obtain output results of the machine learning model as predicted test results for the target item

S130

acquiring monitoring indicators based on the actual test results and the predicted test results

S140

determining whether or not the testing instrument is under control, based on the monitoring indicators, and/or determining whether or not to give an alarm prompt indicating that the testing instrument is out of control, based on the monitoring indicators

# Fig. 1

**Description**

CROSS-REFERENCE

This application is filed based on, and claims priority to, PCT International Patent Application No. PCT/CN2023/097857, filed June 01, 2023, the entire disclosure of which is incorporated herein by reference.

TECHNICAL FIELD

The disclosure relates to the field of quality control for in vitro diagnostic instruments, and in particular to a method and a system for patient-based real-time quality control (PBRTQC), and a method for constructing a quality control model for patient-based real-time quality control (PBRTQC).

BACKGROUND

During clinical diagnosis and treatment of diseases, medical laboratory quality directly affects the diagnosis and treatment of the diseases by doctors. High-quality medical laboratory results rely on a complete quality control system for a medical laboratory. Quality control in a medical laboratory includes three aspects: pre-analytical quality control, analytical quality control, and post-analytical quality control. The analytical quality control is the most critical process in a quality management system. The analytical quality control includes internal quality control (IQC). A currently common practice of implementing IQC is as follows: a laboratory technician tests quality control materials at certain frequency, uses statistical theories to calculate and evaluate the reliability of test results, and then observes and eliminates out-of-control factors in the test. However, IQC monitoring performed by using quality control materials exhibits some typical drawbacks: 1) the process of IQC monitoring performed by using quality control materials is not continuous, but IQC monitoring is performed at individual time points to estimate whether the analytical process is out of control; 2) the quality control materials are not patient samples, and therefore, may produce a matrix effect that affects IQC results; and 3) IQC performed by using quality control materials requires additional costs such as quality control materials, reagents, and human resources, and is thus costly.

SUMMARY

To at least partially solve the above-mentioned technical problems, an objective of the disclosure is to provide an improved PBRTQC-based technical solution that allows for more stable and sensitive internal quality control.
To achieve the above-mentioned objective of the disclosure, a first aspect of the disclosure provides a method for patient-based real-time quality control. The method includes:

> while a testing instrument performs real-time tests on current patient samples for a target item, acquiring actual test results of the current patient samples obtained by the testing instrument for the target item and patient information of the current patient samples, wherein the patient information includes at least one of patient's disease, patient's age, patient's gender, or ordering department for the target item;
> quantifying the patient information of the current patient samples and inputting the quantified patient information into a pre-constructed machine learning model to obtain output results of the machine learning model as predicted test results for the target item, wherein the machine learning model is trained by using actual test results for the target item and patient information of a plurality of historical patient samples;
> acquiring monitoring indicators based on the actual test result and the predicted test result of the current patient samples; and
> determining whether or not the testing instrument is under control based on the monitoring indicators, and, if it is determined that the testing instrument is not under control, outputting an alarm prompt indicating as such.

To achieve the above-mentioned objective of the disclosure, a second aspect of the disclosure provides a computer system for patient-based real-time quality control. The computer system includes:

> a data acquisition module, configured to, while a testing instrument performs real-time tests on current patient samples for a target item, acquire actual test results of the current patient samples obtained by the testing instrument for the target item and patient information of the current patient samples, wherein the patient information includes at least one of patient's disease, patient's age, and patient's gender, or ordering department for the target item;
> a predicted result acquisition module, configured to quantify the patient information of the current patient samples and input the quantified patient information into a pre-constructed machine learning model to obtain output results of the machine learning model as a predicted test result for the target item, wherein the machine learning model is trained by using actual test results for the target item and patient information of a plurality of historical patient samples;
a monitoring indicator acquisition module, configured to acquire monitoring indicators based on the actual test result and the predicted test result of the current patient samples; and
a quality control module, configured to determine whether or not the testing instrument is under control based on the monitoring indicators, and, if it is determined that the testing instrument is not under control, output an alarm prompt indicating as such.

To achieve the above-mentioned objective of the disclosure, a third aspect of the disclosure provides a method for constructing a quality control model for patient-based real-time quality control. The method includes:

acquiring data of a plurality of patient samples and dividing the data into a training set and a test set, the data including actual test results for a target item and patient information of the plurality of patient samples, wherein the actual test results are obtained by a testing instrument performing tests on the patient samples, and the patient information includes at least one of patient's disease, patient's age, patient's gender, or ordering department for the target item, wherein the data in the training set is obtained when the testing instrument is in a controlled state;
building a machine learning model by using the training set by way of quantifying patient information of patient samples in the training set, and building a machine learning model between the quantified patient information and the actual test results in the training set, wherein the machine learning model satisfies the following formula:

$$X_t = f(x_{1t}, x_{2t}, \cdots, x_{nt}) + \varepsilon_t$$

where $X_t$ is the actual test results in the training set, $f(x_{1t}, x_{2t} \cdots, x_{nt})$ is the machine learning model, $x_{1t}, x_{2t}, \cdots, x_{nt}$ represents the quantified patient information, and $\varepsilon_t$ represents a residual of estimation of $X_t$ using the machine learning model;
constructing, based on the machine learning model, a quality control model for patient-based real-time quality control; and
verifying the quality control model by using the test set.

In the technical solutions provided in various aspects of the disclosure, a machine learning model, which is trained based on actual test results and patient information of historical patient samples, is used for acquiring monitoring indicators for quality control of a testing instrument. This can not only overcome the drawbacks of using quality control materials for quality control, but can also realize more stable and more sensitive internal quality control, compared with PBRTQC of the prior art.

BRIEF DESCRIPTION OF THE DRAWINGS

FIG. 1 is a schematic flowchart of a method for patient-based real-time quality control according to an embodiment of the disclosure.
FIG. 2 is a schematic diagram of acquiring the number of samples until error detection.
FIG. 3 is a schematic diagram of monitoring indicators obtained according to an embodiment of the disclosure and monitoring indicators obtained according to the prior art.
FIG. 4 is a schematic block diagram of a computer system for patient-based real-time quality control according to an embodiment of the disclosure.
FIG. 5 is a schematic flowchart of a method for constructing a quality control model for patient-based real-time quality control according to an embodiment of the disclosure.
FIG. 6 is a schematic structural diagram of a neural network model according to an embodiment of the disclosure.
FIG. 7 is a schematic flowchart of a method for optimizing a quality control model according to an embodiment of the disclosure.
FIG. 8 is a schematic flowchart of another method for optimizing a quality control model according to an embodiment of the disclosure.
FIG. 9 is a schematic flowchart of still another method for optimizing a quality control model according to an embodiment of the disclosure.

DESCRIPTION OF THE PREFERRED EMBODIMENTS

The technical solutions of the embodiments of the disclosure will be described below clearly and comprehensively with reference to accompanying drawings of the embodiments of the disclosure. Apparently, the embodiments described are merely some of, rather than all of, the embodiments of the disclosure. Based on the embodiments of the disclosure, all the other embodiments which would have been obtained by those of ordinary skill in the art without any creative efforts shall fall within the scope of protection of the disclosure.

In recent years, patient-based real-time quality control (PBRTQC), due to its characteristics, is able to compensate for some drawbacks of current IQC. The advantages of PBRTQC are as follows: 1) it can use patient samples to monitor the system in real time, so as to detect an instrument out-of-control state in a timely manner; 2) since the patient samples are used, there is no impact of matrix effect; 3) PBRTQC is an analysis software that does not require additional quality control materials, reagents, or labor cost, and is thus less costly.

With the deepening of research and application of PBRTQC, it has been observed that the existing PBRTQC exhibits insufficient error-detection performance for some routine metrics and thus cannot satisfactorily meet clinical IQC requirements.

For example, for patients with diseases, parameter results fluctuate significantly, and the existing PBRTQC requires a large number of consecutive samples for identifying an error and a system out-of-control state, which cannot meet the clinical IQC requirements. In particular, the inventors of the disclosure have found that, for routine metrics such as white blood cell count, red blood cell count, hemoglobin content, thyroid stimulating hormone, etc. factors such as patient's disease, patient's age, and patient's gender cause wide distribution and high fluctuation of the parameter metrics, thereby resulting in a large average number of samples until error detection required for a system based on the existing PBRTQC method.

In view of this, the disclosure provides a technical solution for real-time quality control based on patient samples and a neural network, so as to reduce the impact of factors such as patient's disease, patient's age, and patient's gender on the PBRTQC quality control performance (that is, to improve quality control stability), while reducing the number of samples until error detection compared with the existing PBRTQC quality control (that is, to increase sensitivity).

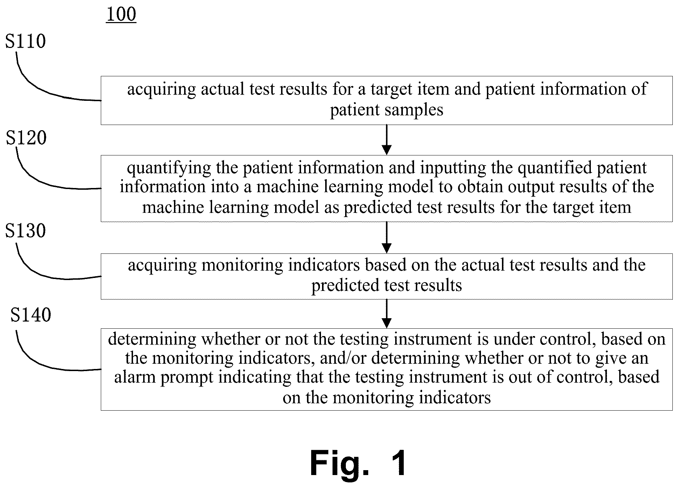

FIG. 1 shows a method 100 for patient-based real-time quality control according to an embodiment of the disclosure. As shown in FIG. 1, the method 100 includes a data acquisition step S110, a data processing step S120, a monitoring indicator acquisition step S130, and a quality control step S140.

In the data acquisition step S110, while a testing instrument performs real-time tests on current patient samples for a target item, in other words, in real time, actual test results of the current patient samples obtained by the testing instrument for the target item and patient information of the current patient samples are acquired, wherein the patient information includes at least one of patient's disease, patient's age, patient's gender, or department to which the respective sample belongs.

In an embodiment of the disclosure, the department to which the respective sample belongs means an ordering department for the target item, that is, a hospital department that requests the use of the testing instrument to test the respective current patient sample for the target item.

In the data processing step S120, the patient information of the current patient samples is quantified, and the quantified patient information is inputted into a pre-constructed machine learning model to obtain output results of the machine learning model as predicted test results for the target item, wherein the machine learning model is trained by using actual test results for the target item and patient information of a plurality of known patient samples (that is, historical patient samples). Here, the machine learning model provided in the embodiment of the disclosure characterizes a relationship between the patient information and the test results for the target item. In other words, if the patient information is inputted into the machine learning model, the predicted test results for the target item predicted by the machine learning model will be obtained.

In the monitoring indicator acquisition step S130, monitoring indicators are acquired based on the actual test results obtained in step S110 and the predicted test results obtained in step S120 of the current patient samples.

In the quality control step S140, it is determined based on the monitoring indicators whether or not the testing instrument is under control, and/or it is determined based on the monitoring indicators whether or not to give an alarm prompt indicating that the testing instrument is not under control. For example, when it is determined based on the monitoring indicators that the testing instrument is not under control, that is, out of control, an alarm prompt indicating that the testing instrument is out of control is outputted.

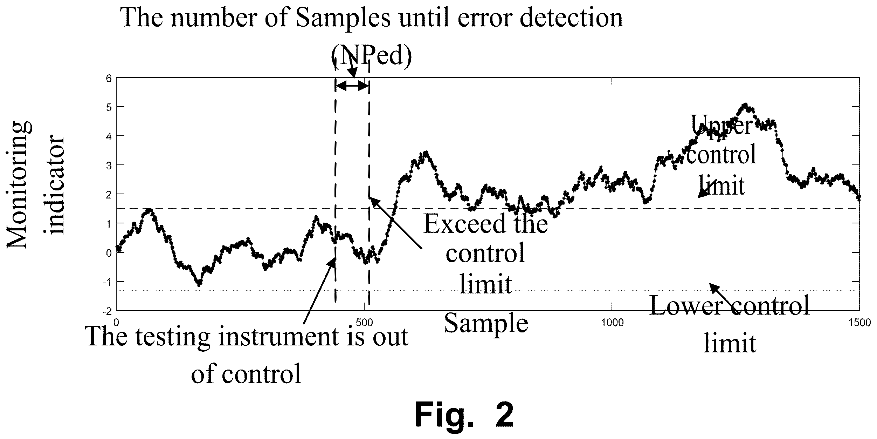

The performance of the PBRTQC-based quality control method of the disclosure and the performance of the PBRTQC-based quality control method of the prior art are verified by using the number of samples required for error detection (also known as the number of patients until error detection, NPed) at the same false alarm rate. The smaller the number of samples required for error detection, the better the sensitivity performance of the error detection. The false alarm rate (FAR) means the number of false alarms triggered by monitoring indicators calculated based on the data obtained by the testing instrument in a controlled state (the number of samples required for the monitoring indicators exceeding a preset control limit) / the number of all samples * 100%. The number of samples required for error detection NPed means the number of samples required from occurrence of an out-of-control event of the testing instrument until a monitoring indicator exceeding the preset control limit appears, as shown in FIG. 2.

Taking that the target items are white blood cell count WBC, red blood cell count RBC, hemoglobin content HGB, and thyroid stimulating hormone TSH as examples for verification. The PBRTQC-based quality control method of the disclosure and the PBRTQC-based quality control method of the prior art are simulated by way of repeatedly adding errors to a WBC verification data set, an RBC verification data set, an HGB verification data set, and a TSH verification data set that are obtained by a respective testing instrument in a controlled state, so as to obtain the average number of samples required for error detection (also known as the average number of patients until error detection) ANPed shown in Table 1. As shown in Table 1, the PBRTQC-based quality control method disclosed in the disclosure significantly improves the quality control sensitivity in contrast to the prior art.

Table 1 ANPed for different target items

| Target item | PBRTQC of the prior art | PBRTQC of the disclosure |
| --- | --- | --- |
| WBC | 229 | 89 |
| RBC | 170 | 91 |
| HGB | 181 | 81 |
| TSH | 410 | 116 |

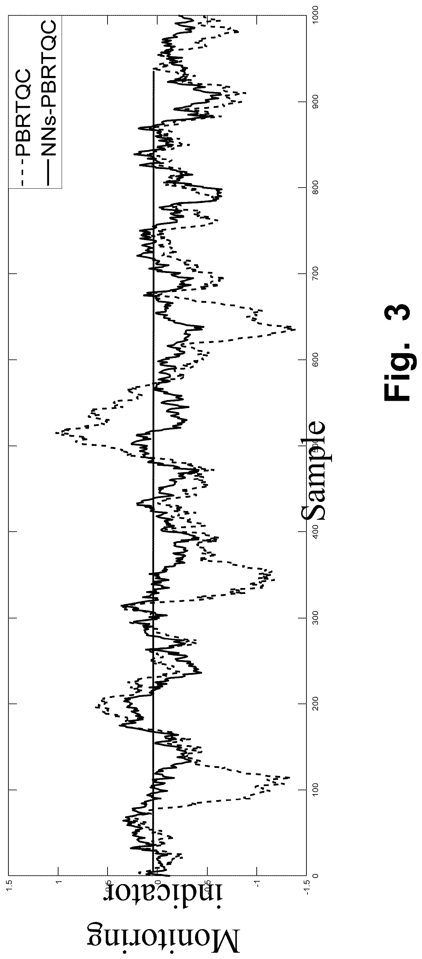

Furthermore, in the case where the alarm rate is the same and the target item is white blood cell count (WBC) as an example, the monitoring indicators NNs-PBRTQC of an embodiment of the disclosure and the monitoring indicators PBRTQC of the existing PBRTQC are calculated respectively based on the WBC dataset obtained by the respective testing instrument in a controlled state, as shown in FIG. 3. As can be seen from FIG. 3, the monitoring indicators calculated according to an embodiment of the disclosure is more stable than the monitoring indicators calculated according to the existing PBRTQC. In addition, at the same alarm rate, the control limit obtained according to an embodiment of the disclosure is smaller than the control limit obtained according to the existing PBRTQC.

In an embodiment of the disclosure, the testing instrument being under control means that the testing instrument is in a normal state and has no fault; and the testing instrument being out of control (namely being not under control) means that the testing instrument is in an abnormal state and may have a fault.

In some embodiments, the machine learning model may be a neural network model. In some other embodiments, the machine learning model may be a support vector machine (SVM)- and/or linear discriminant analysis (LDA)-based machine learning model.

In some embodiments, the target item may be a routine blood test item, for example, cell count (such as white blood cell count, platelet count, red blood cell count) and classification (such as white blood cell classification), and hemoglobin content. Accordingly, the testing instrument is a blood cell analyzer.

In some other embodiments, the target item may be a biochemical test item, for example, thyroid function (for example, thyroid stimulating hormone), liver function, kidney function, blood lipids, blood glucose; Accordingly, the testing instrument is a biochemical analyzer.

In some embodiments, the patient information may include multiple of patient's disease, patient's age, patient's gender, or department to which the respective sample belongs. Preferably, the patient information may include patient's disease, patient's age, patient's gender, and department to which the respective sample belongs.

In some embodiments, the patient information may be acquired from a laboratory (clinical laboratory department) information system of the hospital in which the testing instrument is located.

In some embodiments, the step S130 of acquiring monitoring indicators based on the actual test result and the predicted test result may include:

calculating differences between the actual test results and the predicted test results; and
inputting the differences into a statistical process control (SPC) algorithm-based computational model, to obtain output results of the computational model as the monitoring indicators.

The statistical process control SPC algorithm may include, for example, at least one of moving average, moving median, exponentially weighted moving average, moving standard deviation, moving quantile, or moving sum of outliers.

In some other embodiments, alternatively, the step S130 of acquiring monitoring indicators based on the actual test results and the predicted test results may include acquiring the monitoring indicators based on ratios of the actual test results to the predicted test results.

In some embodiments, quantifying the patient information of the current patient samples, may include: respectively quantifying at least one of the patient information of the current patient samples, especially at least one of patient's disease, patient's gender, or department to which the respective sample belongs, as a matrix.

In an example, respectively quantifying the at least one of the patient information of the current patient samples as a matrix, may include: quantifying each of the at least one of the patient information of the current patient samples as a one-dimensional matrix with a plurality of elements, wherein each element of the one-dimensional matrix represents one category, and said each of the patient information belongs to at least one of the categories represented by the elements.

For example, when the patient information includes disease information, if diseases are classified into 33 disease categories, the disease information may be quantified as a one-dimensional matrix with 33 elements, wherein each element represents one disease category.

For another example, when the patient information includes gender information, gender is divided into 2 categories, i.e., male and female, and therefore the gender information may be quantified as a one-dimensional matrix with 2 elements, in which one element represents male, and the other element represents female.

For still another example, when the patient information includes information about department to which the respective sample belongs, if the department to which the respective sample belongs includes 32 department categories, the information about the department to which the respective sample belongs may be quantified as a one-dimensional matrix with 32 elements, wherein each element represents one department category.

Preferably, quantifying each of the at least one of the patient information of the current patient samples as the one-dimensional matrix with a plurality of elements, may include: quantifying each of the at least one of the patient information of the current patient samples as a one-dimensional matrix with a plurality of elements that is composed of 0s and 1s, wherein a value of the element representing the category to which said each of the patient information belongs is 1, and values of remaining elements are 0. This simplifies the construction of the machine learning model.

For example, when the patient information includes disease information, if diseases are classified into 33 disease categories, the disease information may be quantified as a one-dimensional matrix with 33 elements, $[X_1, X_2, ..., X_{33}]$, wherein each element X represents one disease category, for example, $X_1$ represents hyperthyroidism, $X_2$ represents hypertension, and so on. If the disease information of a current patient sample is hyperthyroidism, the disease information of this patient sample is quantified as [1,0,0,0,...,0], namely the value of element $X_1$ is 1, and the values of the remaining elements are 0. If the disease information of a current patient sample includes hyperthyroidism and hypertension, the disease information of this patient sample is quantified as [1,1,0,0,...,0], namely the values of elements $X_1$ and $X_2$ are 1, and the values of the remaining elements are 0.

For another example, when the patient information includes gender information, gender is divided into 2 categories, i.e., male and female, and therefore the gender information may be quantified as a one-dimensional matrix with 2 elements $[Y_1, Y_2]$, in which the element $Y_1$ represents male, and the element $Y_2$ represents female. If the gender information of a patient sample is male, then the gender information of this patient sample is quantified as [1,0], namely the value of the element $Y_1$ is 1, and the value of the element $Y_2$ is 0.

For still another example, when the patient information includes information about department to which the respective sample belongs, if the department to which the respective sample belongs includes 32 department categories, the information about department to which the respective sample belongs may be quantified as a one-dimensional matrix with 32 elements, $[Z_1, Z_2, ..., Z_{32}]$, wherein each element represents one department category, for example, $Z_1$ represents endocrinology department, $Z_2$ represents nephrology department, and so on. If the information about department to which a patient sample belongs is endocrinology department, the information about the department to which the patient sample belongs is quantified as [1,0,0,0,..., 0], namely the value of the element $Z_1$ is 1, and the values of the remaining elements are 0.

In some other embodiments, quantifying the patient information of the current patient samples, may include: respectively quantifying at least one of the patient information of the current patient samples, especially patient's age, as a fixed value, preferably as a fixed integer.

In an example, patient's age may be quantified as a fixed value of 0 to 150. To be specific, if a patient is 30 years old, then the age of this patient is quantified as 30. In other examples, patient's age may be quantified as a percentage. For example, if a patient is 30 years old, then the age of this patient is quantified as 30/100.

In an example, patient's gender may be quantified as a first positive integer or a second positive integer, wherein the first positive integer represents male, and the second positive integer represents female. For example, the first positive integer is 1, and the second positive integer is 2, but the disclosure is not limited to the example.

Alternatively or additionally, at least one of the patient information of the current patient samples may be quantified through a lookup table method. This simplifies the construction of the machine learning model.

In an example, different positive integers may be set for various corresponding diseases, and the correspondence between each disease and the corresponding positive integer is pre-stored in the form of a lookup table. Therefore, during quantification of disease of a patient, the lookup table is searched for the positive integer corresponding to the disease of this patient. Table 2 shows an example of the lookup table used for quantifying disease.

Table 2 Lookup table for quantifying disease

| Clinical diagnosis | Value |
|---|---|
| Hypertension | 1 |
| Diabetes | 2 |
| Medical examination | 3 |
| Uremia; Peritoneal dialysis therapy | 4 |
| Prostate cancer | 5 |
| ... | ... |

In an example, different positive integers may be set for various corresponding departments, and the correspondence between each department and the corresponding positive integer is pre-stored in the form of a lookup table. Therefore, during quantification of the department to which a patient sample belongs, the lookup table is searched for the positive integer corresponding to the department to which this patient sample belongs. Table 3 shows an example of the lookup table used for quantifying department.

Table 3 Lookup table for quantifying department

| Department | Value |
|---|---|
| Radiation therapy outpatient department | 1 |
| Chemotherapy outpatient department | 2 |
| Infectious diseases outpatient department | 3 |
| Hematology outpatient department | 4 |
| Gynecology outpatient department | 5 |
| ... | ... |

In some embodiments, in step S120, an average of the actual test results for the target item of n patient samples (n being a value such as a daily test flux of the testing instrument) prior to a specified time point among the plurality of known patient samples may be used as a variable of the machine learning model, so as to reduce the daytime impact caused by factors such as calibration of the testing instrument and the daytime impact caused by reagents.

The machine learning model is trained by using the actual test results for the target item and the patient information of the plurality of known patient samples (that is, historical patient samples). Here, the machine learning model may further characterize relationships between the patient information, the mean value of the actual test results of the plurality of known patient samples, and the test results for the target item. In other words, if the patient information is inputted into the machine learning model, a predicted test result for the target item predicted by the machine learning model will be obtained.

In some embodiments, in step S120, that is, acquiring monitoring indicators based on the actual test result and the predicted test result, may include:

performing data processing on the actual test results, wherein the data processing includes truncation processing and/or normalization; and
acquiring the monitoring indicators based on the actual test results that are subjected to the data processing and the predicted test results.

However, in an embodiment of the disclosure, since the machine learning model is used, normalization may not be performed on the actual test results. This simplifies data processing.

In some embodiments, in step S140, it may be determined that the testing instrument is not under control and an alarm prompt may be outputted, when one of the monitoring indicators exceeds a preset control limit.

In some other embodiments, in step S140, it may be determined that the testing instrument is not under control and an alarm prompt may be outputted, only when M consecutive monitoring indicators (successively obtained in chronological order) exceed the preset control limit, where M is a natural number greater than 1. This can reduce the false alarm rate in contrast to the case where an alarm is triggered when one monitoring indicator exceeds the preset control limit.

In still other embodiments, in step S140, monitoring indicators of a consecutive first number M of current patient samples may be successively acquired in chronological order, and it may be determined whether or not the testing instrument is under control based on the monitoring indicators of the consecutive first number M of current patient samples; and it may be determined that the testing instrument is not under control and an alarm prompt indicating as such may be outputted, only when, among the monitoring indicators of the consecutive first number M of current patient samples (successively obtained in chronological order), there at least exist monitoring indicators of a second number N of current patient samples that exceed the preset control limit, where the first number M and the second number N are both natural numbers greater than 1, and the first number M is greater than the second number N. In this way, false alarms can be significantly distinguished from true alarms. This can reduce the false alarm rate and minimize the number of samples required for error detection, in contrast to the case where an alarm is triggered when one monitoring indicator exceeds the preset control limit.

In some embodiments, in step S140, when it is determined that the testing instrument is not under control based on the monitoring indicators, a quality control material for the target item may be automatically dispatched to the testing instrument for testing, to acquire a test result of the quality control material; and it is determined whether the testing instrument is out of control based on the test result of the quality control material. In other words, when it is determined that the testing instrument is not under control based on the test results of the actual patient samples, the quality control material is further used to test the testing instrument, so as to determine whether the testing instrument is truly out of control.

In some embodiments, parameters of the computational model may be optimized by:

acquiring actual test results for the target item and patient information of a plurality of historical patient samples, wherein the actual test results are obtained by the testing instrument performing tests on the plurality of historical patient samples in a controlled state;
setting a false alarm rate;
setting SPC parameter values for the computational model;
determining, at the false alarm rate, an upper control limit and a lower control limit and an average number of samples until error detection, for each SPC parameter value by:

calculating monitoring indicators for said SPC parameter value in the computational model with said SPC parameter value by using the actual test results and the patient information of the plurality of historical patient samples, and acquiring the upper and lower control limits based on the monitoring indicators for said SPC parameter value and based on the false alarm rate, and
calculating monitoring indicators for said SPC parameter value with occurrence of an instrument out-of-control event in the computational model with said SPC parameter value by using a simulated data set including multiple occurrences where the instrument is simulated to be not under control or a real data set including multiple occurrences where the instrument is not under control in reality, and comparing the calculated monitoring indicators with the upper and lower control limits, so as to calculate an average of a number of samples required for each occurrence from when the instrument is simulated to begin to be not under control or the instrument begins to be not under control in reality to when it is actually detected that the instrument is not under control, as the average number of samples until error detection,
wherein the simulated data set is obtained by repeatedly adding deviations to the actual test results of the plurality of historical patient samples at different time points, and the real data set is obtained from the actual test results and the patient information of the plurality of historical patient samples or obtained by acquiring actual test results for the target item and patient information of a plurality of additional historical patient samples; and

selecting an SPC parameter value with a minimum average number of samples until error detection and upper and lower control limits corresponding to the selected SPC parameter value, to construct an optimized computational model.

In some other embodiments, parameters of the truncation processing and parameters of the computational model may be optimized by:

acquiring actual test results for the target item and patient information of a plurality of historical patient samples, wherein the actual test results are obtained by the testing instrument performing tests on the historical patient samples in a controlled state;
setting a false alarm rate;
setting truncation ratios for the truncation processing and SPC parameter values for the computational model;
determining, at the false alarm rate, an upper control limit and a lower control limit and an average number of samples until error detection, for each parameter value combination of each truncation ratio and each SPC parameter value by:

calculating monitoring indicators for said parameter value combination in the computational model with said parameter value combination by using the actual test results and the patient information of the plurality of historical patient samples, and acquiring the upper and lower control limits based on the monitoring indicators for said parameter value combination and based on the false alarm rate, and

calculating monitoring indicators for said parameter value combination with occurrence of an instrument out-of-control event in the computational model with said parameter value combination by using a simulated data set including multiple occurrences where the instrument is simulated to be not under control or a real data set including multiple occurrences where the instrument is not under control in reality, and comparing the calculated monitoring indicators with the upper and lower control limits, so as to calculate an average of a number of samples required for each occurrence from when the instrument is simulated to begin to be not under control or the instrument begins to be not under control in reality to when it is actually detected that the instrument is not under control, as the average number of samples until error detection,

wherein the simulated data set is obtained by repeatedly adding deviations to the actual test results of the plurality of historical patient samples at different time points, and the real data set is obtained from the actual test results and the patient information of the plurality of historical patient samples or obtained by acquiring actual test results for the target item and patient information of a plurality of additional historical patient samples; and

selecting a parameter value combination with a minimum average number of samples until error detection and upper and lower control limits corresponding to the selected parameter value combination, so as to construct an optimized truncation processing and an optimized computational model.

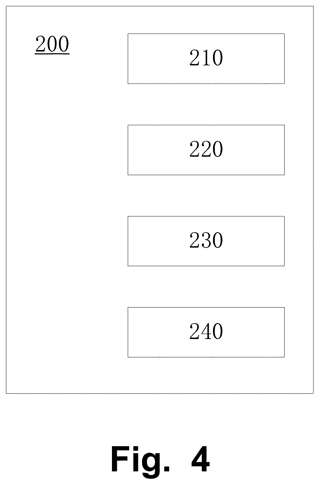

FIG. 4 is a schematic block diagram of a computer system 200 for patient-based real-time quality control according to an embodiment of the disclosure. As shown in FIG. 4, the computer system 200 includes a data acquisition module 210, a predicted result acquisition module 220, a monitoring indicator acquisition module 230, and a quality control module 240.

The data acquisition module 210 is configured to, while a testing instrument performs real-time tests on current patient samples for a target item, acquire in real time actual test results of the current patient samples obtained by the testing instrument for the target item and patient information of the current patient samples, wherein the patient information includes at least one of patient's disease, patient's age, patient's gender, or department to which the respective sample belongs.

The predicted result acquisition module 220 is configured to quantify the patient information of the current patient samples and input the quantified patient information into a pre-constructed machine learning model to obtain output results of the machine learning model as predicted test results for the target item, wherein the machine learning model is trained by using actual test results for the target item and patient information of a plurality of known patient samples.

The monitoring indicator acquisition module 230 is configured to acquire monitoring indicators based on the actual test results and the predicted test results of the current patient samples.

The quality control module 240 is configured to determine whether or not the testing instrument is under control based on the monitoring indicators, and/or to determine whether or not to give an alarm prompt indicating that the testing instrument is not under control based on the monitoring indicators. When it is determined that the testing instrument is not under control based on the monitoring indicators, the quality control module 240 outputs an alarm prompt indicating that the testing instrument is not under control.

The computer system 200 provided in the embodiment of the disclosure may be implemented in the form of hardware, for example, in the form of a processor, or may be implemented in the form of software, for example, software that can be installed and executed on a computer. This is not specifically limited in the disclosure.

In some embodiments, the machine learning model may be a neural network model. In some other embodiments, the machine learning model may be a support vector machine (SVM)- and/or linear discriminant analysis (LDA)-based machine learning model.

In some embodiments, the target item may be a routine blood test item, for example, cell count (such as white blood cell count, platelet count, red blood cell count) and classification (such as white blood cell classification), and hemoglobin content. Accordingly, the testing instrument is a blood cell analyzer.

In some other embodiments, the target item may be a biochemical test item, for example, thyroid function (for example, thyroid stimulating hormone), liver function, kidney function, blood lipids, blood glucose. Accordingly, the testing instrument is a biochemical analyzer.

In some embodiments, the patient information may include multiple of patient's disease, patient's age, patient's gender, or department to which the respective sample belongs. Preferably, the patient information may include patient's disease, patient's age, patient's gender, and department to which the respective sample belongs.

In some embodiments, the patient information may be acquired from a laboratory (clinical laboratory department) information system of the hospital in which the testing instrument is located.

In some embodiments, the monitoring indicator acquisition module 230 may be further configured to:

calculate differences between the actual test results and the predicted test results; and
input the differences into a statistical process control (SPC) algorithm-based computational model, to obtain output results of the computational model as the monitoring indicators.

The process control SPC algorithm may include, for example, at least one of moving average, moving median, exponentially weighted moving average, moving standard deviation, moving quantile, or moving sum of outliers.

In some embodiments, the predicted result acquisition module 220 may be further configured to respectively quantify at least one of the patient information of the current patient samples, especially at least one of patient's disease, patient's gender, or department to which the respective sample belongs, as a matrix.

Further, the predicted result acquisition module 220 may be further configured to quantify each of the at least one of the patient information of the current patient samples as a one-dimensional matrix with a plurality of elements, wherein each element of the one-dimensional matrix represents one category, and said each of the patient information belongs to at least one of categories represented by the elements.

Preferably, the predicted result acquisition module 220 may be further configured to quantify each of the at least one of the patient information of the current patient samples as a one-dimensional matrix with a plurality of elements that is composed of 0s and 1s, wherein a value of the element representing the category to which said each of the patient information belongs is 1, and values of remaining elements are 0.

In some embodiments, the predicted result acquisition module 220 may be further configured to quantify at least one of the patient information of the current patient samples, especially patient's age, as a fixed value, preferably as a fixed integer.

Alternatively or additionally, the predicted result acquisition module 220 may be further configured to quantify at least one of the patient information of the current patient samples through a lookup table method. This simplifies the construction of the machine learning model.

For specific quantification examples, reference may be made to the above detailed description of various embodiments of the method 100, which will not be repeated herein.

In some embodiments, the monitoring indicator acquisition module 230 may be further configured to:

perform data processing on the actual test results, wherein the data processing includes truncation processing and/or normalization; and
acquire the monitoring indicators based on the actual test results that are subjected to the data processing and the predicted test results.

However, in an embodiment of the disclosure, since the machine learning model is used, normalization may not be performed on the actual test results. This simplifies data processing.

In some embodiments, the quality control module 240 may be further configured to determine that the testing instrument is not under control and output an alarm prompt when one of the monitoring indicators exceeds a preset control limit.

In some other embodiments, the quality control module 240 may be further configured to determine that the testing instrument is not under control and output an alarm prompt only when M consecutive monitoring indicators (successively obtained in chronological order) exceed the preset control limit, where M is a natural number greater than 1. This can reduce the false alarm rate in contrast to the case where an alarm is triggered when one monitoring indicator exceeds the preset control limit.

In still other embodiments, the quality control module 240 may be further configured to successively acquire monitoring indicators of a consecutive first number M of the current patient samples in chronological order, and determine whether or not the testing instrument is under control based on the monitoring indicators of the consecutive first number M of the current patient samples; determine that the testing instrument is not under control and output an alarm prompt that indicates the testing instrument is not under control, only when, among the monitoring indicators of the consecutive first number M of current patient samples (successively obtained in chronological order), there at least exist monitoring indicators of a second number N of current patient samples that exceed the preset control limit, where the first number M and the second number N are both natural numbers greater than 1, and the first number M is greater than the second number N. In this way, false alarms can be significantly distinguished from true alarms. This can reduce the false alarm rate and minimize the number of samples until error detection, in contrast to the case where an alarm is triggered when one monitoring indicator exceeds the preset control limit.

In some embodiments, the quality control module 240 may be further configured to, when it is determined that the testing instrument is not under control based on the monitoring indicators, automatically dispatch a quality control material for the target item to the testing instrument for testing, to acquire a test result of the quality control material; and determine whether the testing instrument is out of control based on the test result of the quality control material.

In some embodiments, the monitoring indicator acquisition module may be optimized by:

acquiring actual test results for the target item and patient information of a plurality of historical patient samples, wherein the actual test results are obtained by the testing instrument performing tests on the plurality of historical patient samples in a controlled state;

setting a false alarm rate;

setting SPC parameter values of the computational model;

determining, at the false alarm rate, an upper control limit and a lower control limit and an average number of samples until error detection, for each SPC parameter value by:

calculating monitoring indicators for said SPC parameter value in the computational model with said SPC parameter value by using the actual test results and the patient information of the plurality of historical patient samples, and acquiring the upper and lower control limits based on the monitoring indicators for said SPC parameter value and based on the false alarm rate, and

calculating monitoring indicators for said SPC parameter value with occurrence of an instrument out-of-control event in the computational model with said SPC parameter value by using a simulated data set including multiple occurrences where the instrument is simulated to be not under control or a real data set including multiple occurrences where the instrument is not under control in reality, and comparing the calculated monitoring indicators with the upper and lower control limits, so as to calculate an average of a number of samples required for each occurrence from when the instrument is simulated to begin to be not under control or the instrument begins to be not under control in reality to when it is actually detected that the instrument is not under control, as the average number of samples until error detection,

wherein the simulated data set is obtained by repeatedly adding deviations to the actual test results of the plurality of historical patient samples at different time points, and the real data set is obtained from the actual test results and the patient information of the plurality of historical patient samples or obtained by acquiring actual test results for the target item and patient information of a plurality of additional historical patient samples; and

selecting an SPC parameter value with a minimum average number of samples until error detection and upper and lower control limits corresponding to the selected SPC parameter value, to construct an optimized computational model.

In some other embodiments, the monitoring indicator acquisition module may be optimized by:

acquiring actual test results for the target item and patient information of a plurality of historical patient samples, wherein the actual test results are obtained by the testing instrument performing tests on the historical patient samples in a controlled state;

setting a false alarm rate;

setting truncation ratios for the truncation processing and SPC parameter values of the computational model;

determining, at the false alarm rate, an upper control limit and a lower control limit and an average number of samples until error detection, for each parameter value combination of each truncation ratio and each SPC parameter value by:

calculating monitoring indicators for said parameter value combination in the computational model with said parameter value combination by using the actual test results and the patient information of the plurality of historical patient samples, and acquiring the upper and lower control limits based on the monitoring indicators at the parameter value combination and based on the false alarm rate, and

calculating monitoring indicators for said parameter value combination with occurrence of an instrument out-of-control event in the computational model with said parameter value combination by using a simulated data set including multiple occurrences where the instrument is simulated to be not under control or a real data set including multiple occurrences where the instrument is not under control in reality, and comparing the calculated monitoring indicators with the upper and lower control limits, so as to calculate an average of a number of samples required for each occurrence from when the instrument is simulated to begin to be not under control or the instrument begins to be not under control in reality to when it is actually detected that the instrument is not under control, as the average number of samples until error detection,

wherein the simulated data set is obtained by repeatedly adding deviations to the actual test results of the plurality of historical patient samples at different time points, and the real data set is obtained from the actual test results and the patient information of the plurality of historical patient samples or obtained by acquiring actual test results for the target item and patient information of a plurality of additional historical patient samples; and

selecting a parameter value combination with a minimum average number of samples until error detection and upper and lower control limits corresponding to the selected parameter value combination, so as to construct an optimized truncation processing and an optimized computational model.

The various embodiments of the method 100 provided in the embodiments of the disclosure, and the advantages thereof, may correspondingly apply to the computer system 200 provided in the embodiments of the disclosure, and thus will not be repeated.

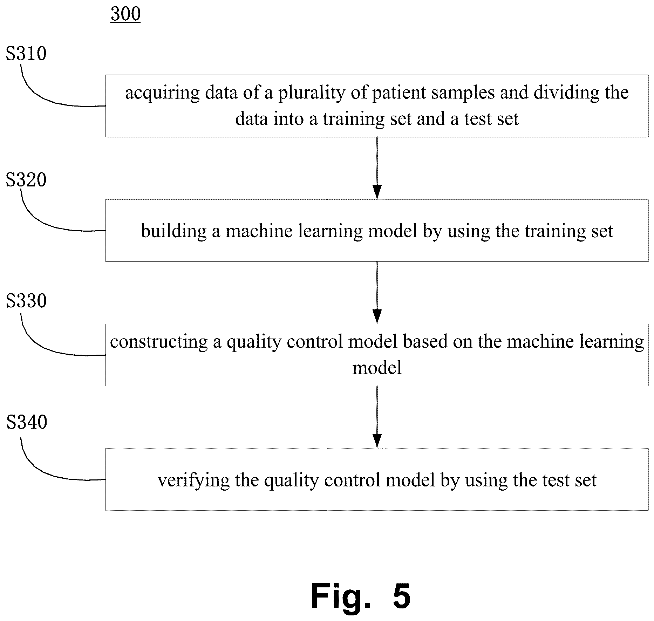

FIG. 5 shows a method 300 for constructing a quality control model for patient-based real-time quality control according to an embodiment of the disclosure. The method includes the following steps:

S310: acquiring data of a plurality of patient samples and divide the data into a training set and a test set (for example, the data can be divided into the training set and the test set according to chronological order), wherein the data includes actual test results for a target item and patient information of the plurality of patient samples, wherein the actual test results are obtained by the testing instrument performing tests on the patient samples, and the patient information includes at least one of patient's disease, patient's age, patient's gender, or department to which the respective sample belongs, wherein the data in the training set is obtained when the testing instrument is in a controlled state. Here, for example, actual test results of a plurality of patient samples, especially all patient samples, which are tested by the testing instrument for the target item in chronological order over a period of time, and patient information thereof, are obtained.

In step S310, the sample ratio of the training set to the test set may be set to 6:4 or 7:3, or the like.

S320: building a machine learning model using the training set by way of quantifying the patient information of the patient samples in the training set, and building a machine learning model between the quantified patient information and the actual test results in the training set, wherein the machine learning model satisfies the following formula:

$$X_t = f(x_{1t}, x_{2t}, \cdots, x_{nt}) + \varepsilon_t$$

where $X_t$ is the actual test results in the training set, $f(x_{1t}, x_{2t}, \cdots, x_{nt})$ is the machine learning model, $x_{1t}, x_{2t}, \cdots, x_{nt}$ represents the quantified patient information, and $\varepsilon_t$ represents a residual estimated by using the machine learning model $X_t$. In other words, the machine learning model f is trained by performing regression learning on historical test results obtained by the testing instrument in a controlled state and patient information, where $\varepsilon_t$ approximately follows a random distribution with a mean value of 0.

S330: constructing, based on the machine learning model, a quality control model for patient-based real-time quality control.

S340: verifying the quality control model by using the test set, so as to verify the performance of the quality control model.

The machine learning model f is an artificial neural network nonlinear function, and reasonably estimates deviations caused by the factors such as disease, age and gender. After such factors affecting parameter fluctuations are eliminated, the monitoring indicators obtained by using the quality control model constructed based on the machine learning model are stable, thereby improving the system error detection performance in contrast to PBRTQC of the prior art.

When the testing instrument is in a normally controlled state, the residual $\varepsilon_t$ fluctuates randomly, and the sum-of-squares error is minimal. In some embodiments, a loss function $min\Sigma\|\varepsilon_t\|^2$ may be created based on this principle. A machine learning model $f(x_{1t}, x_{2t}, \cdots, x_{nt})$ with a minimized loss function is calculated by using training samples (that is, the actual test results and the patient information of the plurality of patient samples) as inputs.

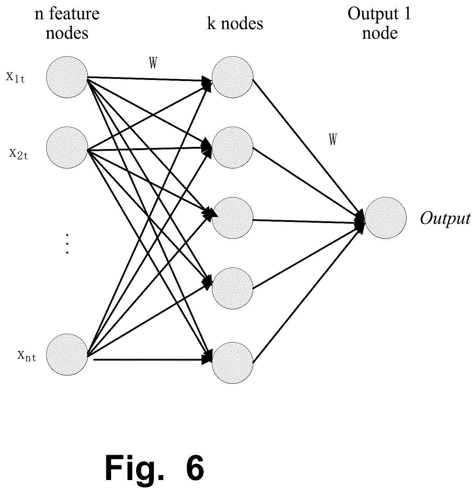

In some embodiments, the machine learning model may be a neural network model, wherein the neural network model is structured as shown in FIG. 6. In some other embodiments, the machine learning model may be a machine learning model based on a support vector machine (SVM) and/or linear discriminant analysis (LDA).

In some embodiments, in step S330, constructing, based on the machine learning model, the quality control model for patient-based real-time quality control, may include: constructing the quality control model based on the machine learning model and a statistical process control (SPC) algorithm-based computational model, wherein differences between the actual test results and the test results predicted by the machine learning model f, that is, residuals $\varepsilon_t$, are inputted into the statistical process control (SPC) algorithm-based computational model, to obtain outputs of the computational model as a monitoring indicators of the quality control model.

However, in other embodiments, the monitoring indicator of the quality control model may be calculated in other ways instead, for example, by calculating ratios between the actual test results and the test results predicted by the machine learning model f.

In some embodiments, in step S320, an average of the actual test results for the target item of n patient samples (n being a value such as a daily test flux of the testing instrument) prior to a specified time point among the plurality of known patient samples may be used as a variable of the machine learning model, so as to reduce the daytime impact caused by factors such as calibration of the testing instrument and the daytime impact caused by reagents.

Further, in step S330, a false alarm rate is set, and an upper control limit and a lower control limit of the quality control model are acquired based on the monitoring indicators and the false alarm rate. Here, while performing real-time quality control by using the quality control model, if the testing instrument is out of control, the residual $\varepsilon_t$ exhibits a non-random feature; after calculation based on a statistical process control (SPC) algorithm, there exist at least one monitoring indicator exceeds the upper control limit or the lower control limit, and an alarm is thus generated, indicating that the testing instrument is out of control.

In some embodiments, the statistical process control SPC algorithm may include at least one of moving mean, moving median, exponentially weighted moving average, moving standard deviation, moving quantile, or moving sum of outliers.

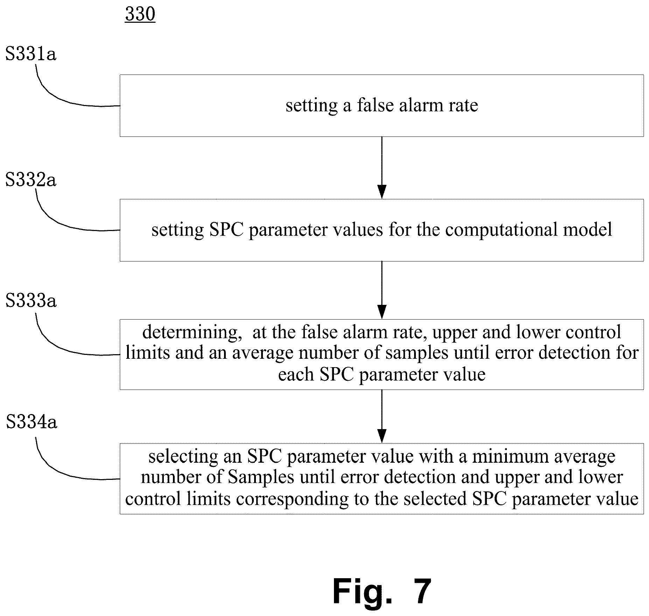

In some embodiments, as shown in FIG. 7, in step S330, parameters of the computational model may be optimized by:

step S331a: setting a false alarm rate FAR;
step S332a: setting SPC parameter values for the computational model;
step S333a: determining, at the false alarm rate, an upper control limit and a lower control limit and an average number of samples until error detection, for each SPC parameter value by:

calculating monitoring indicators for said SPC parameter value in the quality control model with said SPC parameter value by using the actual test results and the patient information in the training set, and acquiring the upper and lower control limits based on the monitoring indicators for said SPC parameter value and based on the false alarm rate, and

calculating monitoring indicators for said parameter value with occurrence of an instrument out-of-control event in the quality control model with said SPC parameter value by using a simulated data set including multiple occurrences where the instrument is simulated to be not under control or a real data set including multiple occurrences where the instrument is not under control in reality, and comparing the calculated monitoring indicators with the upper and lower control limits, so as to calculate an average of a number of samples required for each occurrence from when the instrument is simulated to begin to be not under control or the instrument begins to be not under control in reality to when it is actually detected that the instrument is not under control by the quality control model, as the average number of samples until error detection ANPed, wherein the simulated data set is obtained by repeatedly adding deviations to the actual test results in the training set at different time points, and the real data set is obtained from the data of the plurality of patient samples or obtained by acquiring actual test results for the target item and patient information of a plurality of additional patient samples; and

step S334a: selecting an SPC parameter value with a minimum average number of samples until error detection and upper and lower control limits corresponding to the selected SPC parameter value, so as to construct an optimized quality control model.

Here, the false alarm rate directly determines the upper and lower control limits. The false alarm rate is usually set manually by a user in view of the usage situation. For example, the false alarm rate is set to 0.1%. Once the false alarm rate is determined, the upper and lower control limits can also be determined.

Here, it should be understood that values of other parameters of the quality control model such as the truncation ratio are fixed, for example, are selected empirically, during optimization of the parameters of the computational model.

Preferably, the average number of samples until error detection ANPed is calculated by using a simulated data set including multiple occurrences where the instrument is simulated to be not under control as follows: calculating monitoring indicators for said SPC parameter value in the quality control model with said SPC parameter value by using the actual test results and the patient information in the training set, and acquiring the upper and lower control limits based on the monitoring indicators for said parameter value and based on the false alarm rate; and repeatedly adding, at different time points, deviations to the actual test results for the target item of the patient samples in the training set; after adding the deviations, inputting the actual test results for the target item of the patient samples in the training set together with the corresponding patient information into the quality control model with said SPC parameter value, so as to obtain monitoring indicators after the addition of the deviations, and comparing these monitoring indicators with the upper and lower control limits, so as to calculate an average of a number of samples required from the beginning of the addition of each deviation until the actual detection of said deviation by the quality control model, this is, to calculate the average number of samples until error detection ANPed.

Taking that the statistical process control SPC algorithm is a moving average method as an example, the parameter of the moving average method-based computational model is a sliding window. The false alarm rate is set to 0.1%, and the parameter value of the sliding window is set to 5, 10, 15, 20, 50, and 100. Monitoring indicators for different sliding windows are calculated by using the training set, and upper and lower control limits are then calculated based on the monitoring indicators and the false alarm rate (for example, if the monitoring indicators are calculated by using the actual test results for the target item and the patient information of N patient samples, then the number of false alarms is N*0.1%, and both the number of false alarms below the upper control limit and the number of false alarms above the lower control limit are N*0.1%/2. Accordingly, the upper control limit and the lower control limit are determined, wherein there are respectively N*0.1%/2 monitoring indicators beyond the upper control limit and beyond the lower control limit). Subsequently, deviations are added randomly at a plurality of positions in the training set, a number of samples until error detection for each deviation is calculated, and then the calculated values are averaged out to obtain an average number of samples until error detection, thereby determining a parameter value satisfying the specified false alarm rate and a minimum average number of samples until error detection.

In some embodiments, steps S331a, S332a, S333a, and S334a may be repeated, and then a minimum average number of samples until error detection ANPedmin is selected from the average numbers of samples until error detection for a plurality of false alarm rates FAR (for example, 0.001%, 0.01%, 0.1%, 1%, 3%, and 5%), so as to obtain a parameter value corresponding to the minimum average number of samples until error detection and upper and lower control limits corresponding to the parameter value, so as to construct an optimized quality control model. In other embodiments, a false alarm rate FAR and a minimum average number of samples until error detection ANPedmin at this false alarm rate FAR may also be weighted and then summed, expressed as: sum = FAR * a + ANPedmin * b, where a is the weight of the false alarm rate FAR and b is the weight of the minimum average number of samples until error detection ANPedmin. The sum of each false alarm rate FAR and a corresponding minimum average number of samples until error detection ANPedmin at this false alarm rate FAR is calculated, and then the minimum average number of samples until error detection corresponding to a minimum sum and a corresponding parameter value are selected.

In some other embodiments, if the test set includes data with multiple occurrences where the instrument is not under control in reality, then upper and lower control limits and an average number of samples until error detection at the false alarm rate may be determined for each SPC parameter value by using the test set as the real data set, by way of: calculating monitoring indicators for said SPC parameter value in the quality control model with said SPC parameter value by using the actual test results for the target item and the patient information of the patient samples in the training set, and acquiring the upper and lower control limits based on the monitoring indicators for said SPC parameter value and based on the false alarm rate; and inputting the actual test results for the target item of the patient samples in the test set together with the corresponding patient information into the quality control model with said SPC parameter value, so as to obtain monitoring indicators corresponding to the test set; and comparing these monitoring indicators with the upper and lower control limits, so as to calculate an average of a number of samples required for each occurrence from when the instrument begins to be not under control in reality to when it is actually detected that the instrument is not under control by the quality control model, this is, to calculate the average number of samples until error detection ANPed.

In still other embodiments, another test set may be obtained, wherein the other test set includes actual test results for a target item and patient information of a plurality of patient samples. The actual test results are obtained by the testing instrument testing the patient samples, and the patient information includes at least one of patient's disease, patient's age, patient's gender, or department to which the respective sample belongs. The other test set includes data with multiple occurrences where the instrument is not under control in reality. Here, upper and lower control limits and an average number of samples until error detection at the false alarm rate may be determined for each SPC parameter value by using the other test set, by way of: calculating monitoring indicators for said SPC parameter value in the quality control model with said SPC parameter value by using the actual test results for the target item and the patient information of the patient samples in the training set, and acquiring the upper and lower control limits based on the monitoring indicator for said SPC parameter value and based on the false alarm rate; and inputting the actual test results for the target item of the patient samples in the other test set together with the corresponding patient information into the quality control model with said SPC parameter value, so as to obtain monitoring indicators corresponding to the other test set; and comparing these monitoring indicators with the upper and lower control limits, so as to calculate an average of a number of samples required for each occurrence from when the instrument begins to be not under control in reality to when it is actually detected that the instrument is not under control by the quality control model, this is, to calculate the average number of samples until error detection ANPed.

In some embodiments, truncation processing, and optionally normal transformation, may be performed on the actual test results for the target item of the patient samples in the training set before building the machine learning model.

In some embodiments, a fixed truncation ratio may be set for the truncation processing.

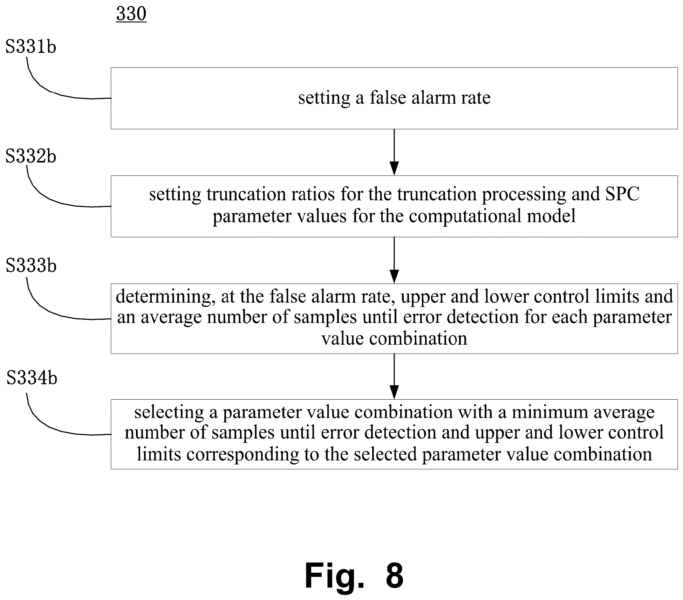

In some embodiments, as shown in FIG. 8, parameters of the truncation processing and parameters of the computational model may be optimized by:

　　step S331b: setting a false alarm rate FAR;

step S332b: setting truncation ratios for the truncation processing and SPC parameter values for the computational model;

step S333b: determining, at the false alarm rate, upper and lower control limits and an average number of samples until error detection, for each parameter value combination of each truncation ratio and each SPC parameter value by:

calculating monitoring indicators for said parameter value combination in the quality control model with said parameter value combination by using the actual test results and the patient information in the training set, and acquiring the upper and lower control limits based on the monitoring indicators for said parameter value combination and based on the false alarm rate, and

calculating monitoring indicators for said parameter value combination with occurrence of an instrument out-of-control event in the quality control model with said parameter value combination by using a simulated data set including multiple occurrences where the instrument is simulated to be not under control or a real data set including multiple occurrences where the instrument is not under control in reality, and comparing the calculated monitoring indicators with the upper and lower control limits, so as to calculate an average of a number of samples required for each occurrence from when the instrument is simulated to begin to be not under control or the instrument begins to be not under control in reality to when it is actually detected that the instrument is not under control by the quality control model, as the average number of samples until error detection,

wherein the simulated data set is obtained by repeatedly adding deviations to the actual test results in the training set at different time points, and the real data set is obtained from the data of the plurality of patient samples or obtained by acquiring actual test results for the target item and the patient information of a plurality of additional patient samples; and

step S334b: selecting a parameter value combination with a minimum average number of samples until error detection and upper and lower control limits corresponding to the selected parameter value combination, so as to construct an optimized quality control model.

Here, it should be understood that values of other parameters of the quality control model are fixed, for example, selected empirically, during optimization of the parameters of the truncation processing and the parameters of the computational model.

Preferably, the average number of samples until error detection ANPed is calculated by using a simulated data set including multiple occurrences where the instrument is simulated to be not under control as follows: calculating monitoring indicators for said parameter value in the quality control model with said parameter value combination by using the actual test results for the target item and the patient information of the patient samples in the training set, and acquiring the upper and lower control limits based on the monitoring indicators for said parameter value and based on the false alarm rate; repeatedly adding, at different time points, deviations to the actual test results for the target item of the patient samples in the training set; after adding the deviations, inputting the actual test results for the target item of the patient samples in the training set together with the corresponding patient information into the quality control model with said parameter value combination, so as to obtain monitoring indicators after the addition of the deviations; and comparing these monitoring indicators with the upper and lower control limits, so as to calculate an average of a number of samples required from the beginning of the addition of each deviation until the actual detection of said deviation by the quality control model, this is, to calculate the average number of samples until error detection ANPed.

Likewise, in some embodiments, steps S331b, S332b, S333b and S334b may be repeated, and then a minimum average number of samples until error detection ANPedmin is selected from the average numbers of samples until error detection for a plurality of false alarm rates FAR (for example, 0.001%, 0.01%, 0.1%, 1%, 3%, and 5%), so as to obtain a parameter value combination corresponding to the minimum average number of samples until error detection and upper and lower control limits corresponding to the parameter value combination, so as to construct an optimized quality control model. In other embodiments, a false alarm rate FAR and a minimum average number of samples until error detection ANPedmin at this false alarm rate FAR may also be weighted and then summed, expressed as: sum = FAR * a + ANPedmin * b, where a is the weight of the false alarm rate FAR and b is the weight of the minimum average number of samples until error detection ANPedmin. The sum of each false alarm rate FAR and a corresponding minimum average number of samples until error detection ANPedmin at this false alarm rate FAR is calculated, and then the minimum average number of samples until error detection corresponding to a minimum sum and a corresponding parameter value combination are selected.

Taking that the statistical process control SPC algorithm is a moving average method as an example, the parameter of the moving average method-based computational model is a sliding window. Parameters to be optimized of the quality control model include a sliding window and a truncation ratio. The false alarm rate is set to 0.1%, the parameter value of the sliding window is set to 5, 10, 15, 20, 50, and 100, and the parameter value of the truncation ratio for the truncation processing is set to $\pm 0\%$, $\pm 1\%$, $\pm 2\%$, and $\pm 5\%$. Monitoring indicators for different parameter value combinations of the sliding window and the truncation ratio (in this example, there are a total of 24 parameter value combinations) are calculated by using the training set, and upper and lower control limits are then calculated based on the monitoring indicators and the false alarm rate (for example, if the monitoring indicators are calculated by using the actual test results for the target item and the patient information of N patient samples, then the number of false alarms is N*0.1%, and both the number of false alarms below the upper control limit and the number of false alarms above the lower control limit are N*0.1%/2. Accordingly, the upper control limit and the lower control limit are determined, wherein there are respectively N*0.1%/2 monitoring indicators beyond the upper control limit and beyond the lower control limit). Subsequently, deviations are added randomly at a plurality of positions in the training set, a number of samples until error detection for each deviation is calculated, and then the calculated values are averaged out to obtain an average number of samples until error detection, thereby determining a parameter value satisfying the specified false alarm rate and a minimum average number of samples until error detection.

In some embodiments, if the test set includes data with multiple occurrences where the instrument is not under control in reality, then upper and lower control limits and an average number of samples until error detection at the false alarm rate may be determined for each parameter value combination by using the test set, by way of: calculating monitoring indicators for said parameter value combination in the quality control model with said parameter value combination by using the actual test results for the target item and the patient information of the patient samples in the training set, and acquiring the upper and lower control limits based on the monitoring indicators for said parameter value combination and based on the false alarm rate, and inputting the actual test results for the target item of the patient samples in the test set together with the corresponding patient information into the quality control model with said parameter value combination, so as to obtain monitoring indicators corresponding to the test set; and comparing these monitoring indicators with the upper and lower control limits, so as to calculate an average of a number of samples required for each occurrence from when the instrument begins to be not under control in reality to when it is actually detected that the instrument is not under control by the quality control model, this is, to calculate the average number of samples until error detection ANPed.

In still other embodiments, another test set may be obtained, wherein the other test set includes actual test results for a target item and patient information of a plurality of patient samples. The actual test results are obtained by the testing instrument testing the patient samples, and the patient information includes at least one of patient's disease, patient's age, patient's gender, or department to which the respective sample belongs. The other test set includes data with multiple occurrences where the instrument is not under control in reality. Here, upper and lower control limits and an average number of samples until error detection at the false alarm rate may be determined for each parameter value combination by using the other test set, by way of: calculating monitoring indicators for said parameter value combination in the quality control model with said parameter value combination by using the actual test results for the target item and the patient information of the patient samples in the training set, and acquiring the upper and lower control limits based on the monitoring indicators for said parameter value combination and based on the false alarm rate, and inputting the actual test results for the target item of the patient samples in the other test set together with the corresponding patient information into the quality control model with said parameter value combination, so as to obtain monitoring indicators corresponding to the other test set; and comparing these monitoring indicators with the upper and lower control limits, so as to calculate an average of a number of samples required for each occurrence from when the instrument begins to be not under control in reality to when it is actually detected that the instrument is not under control by the quality control model, this is, to calculate the average number of samples until error detection ANPed.

In some embodiments, when real-time quality control is performed by using the quality control model, it may be determined that the testing instrument is not under control and an alarm prompt may be outputted only when at least N monitoring indicators among M consecutive monitoring indicators (successively obtained in chronological order) exceed the upper control limit or the lower control limit, where M and N are both natural numbers greater than 1, and M is greater than N.

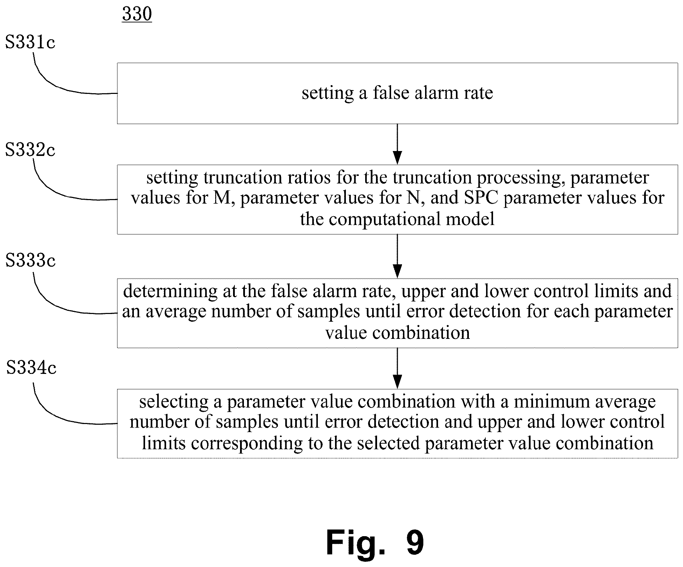

In some embodiments, as shown in FIG. 9, M and N may also be parameters to be optimized of the quality control model, that is, parameters of the computational model, M, N, and optionally parameters of the truncation processing may be optimized by:

> S331c: setting a false alarm rate;
> S332c: setting SPC parameter values for the computational model, parameter values for M, parameter values for N, and optionally truncation ratios for the truncation processing;
> S333c: determining, at the false alarm rate, upper and lower control limits and an average number of samples until error detection for each parameter value combination of each SPC parameter value, each parameter value for M, each parameter value for N, and optionally each truncation ratio by:

>> calculating monitoring indicators for said parameter value combination in the quality control model with said parameter value combination by using the actual test results and the patient information in the training set, and acquiring the upper and lower control limits based on the monitoring indicators for said parameter value combination and based on the false alarm rate, and

calculating monitoring indicators for said parameter value combination with occurrence of an instrument out-of-control event in the quality control model with said parameter value combination by using a simulated data set including multiple occurrences where the instrument is simulated to be not under control or a real data set including multiple occurrences where the instrument is not under control in reality, and comparing the calculated monitoring indicators with the upper and lower control limits, so as to calculate an average of a number of samples required for each occurrence from when the instrument is simulated to begin to be not under control or the instrument begins to be not under control in reality to when it is actually detected that the instrument is not under control by the quality control model, as the average number of samples until error detection,

wherein the simulated data set is obtained by repeatedly adding deviations to the actual test results in the training set at different time points, and the real data set is obtained from the data of the plurality of patient samples or obtained by acquiring actual test results for the target item and the patient information of a plurality of additional patient samples; and

S334c: selecting a parameter value combination with a minimum average number of samples until error detection and upper and lower control limits corresponding to the selected parameter value combination, so as to construct an optimized quality control model.

Here, it should be understood that values of other parameters of the quality control model are fixed, for example, selected empirically, during optimization of the parameters of the computational model, M, N, and optionally the parameters of the truncation processing. For example, the truncation ratio for the truncation processing may be fixed during optimization of the parameters of the computational model, M, and N.

Likewise, in some embodiments, steps S331c, S332c, S333c and S334c may be repeated, and then a minimum average number of samples until error detection ANPedmin is selected from the average numbers of samples until error detection for a plurality of false alarm rates FAR (for example, 0.001%, 0.01%, 0.1%, 1%, 3%, and 5%), so as to obtain a parameter value combination corresponding to the minimum average number of samples until error detection and upper and lower control limits corresponding to the parameter value combination, so as to construct an optimized quality control model. In other embodiments, a false alarm rate FAR and a minimum average number of samples until error detection ANPedmin at this false alarm rate FAR may also be weighted and then summed, expressed as: sum = FAR * a + ANPedmin * b, where a is the weight of the false alarm rate FAR and b is the weight of the minimum average number of samples until error detection ANPedmin. The sum of each false alarm rate FAR and a corresponding minimum average number of samples until error detection ANPedmin at this false alarm rate FAR is calculated, and then the minimum average number of samples until error detection corresponding to a minimum sum and a corresponding parameter value combination are selected.

Preferably, the average number of samples until error detection ANPed is calculated by using a simulated data set including multiple occurrences where the instrument is simulated to be not under control as follows: calculating monitoring indicators for said parameter value combination in the quality control model with said parameter value combination by using the actual test results and the patient information in the training set, and acquiring the upper and lower control limits based on the monitoring indicators for said parameter value combination and based on the false alarm rate; and repeatedly adding, at different time points, deviations to the actual test results for the target item of the patient samples in the training set; after adding the deviations, inputting the actual test results for the target item of the patient samples in the training set together with the corresponding patient information into the quality control model with said parameter value combination, so as to obtain monitoring indicators after the addition of the deviations; and comparing these monitoring indicators with the upper and lower control limits, so as to calculate the average number of samples required from the beginning of the addition of each deviation until the actual detection of said deviation by the quality control model, this is, to calculate the average number of samples until error detection ANPed.

Taking that the statistical process control SPC algorithm is a moving average method as an example, the parameter of the moving average method-based computational model is a sliding window. Parameters to be optimized of the quality control model include a sliding window, a truncation ratio, M, and N. The false alarm rate is set to 0.1%, the parameter value of the sliding window is set to 10, 20, 50, 70, and 100, the parameter value of the truncation ratio for the truncation processing is set to $\pm0\%$, $\pm1\%$, $\pm2\%$, and $\pm5\%$, and the parameter values for M and N are set according to Table 4. Monitoring indicators for different parameter value combinations of the sliding window, the truncation ratio, M, and N are calculated by using the training set, and upper and lower control limits are then calculated based on the monitoring indicators and the false alarm rate. Subsequently, deviations are added randomly at a plurality of positions in the training set, a number of samples until error detection for each deviation is calculated, and then the calculated values are averaged out to obtain an average number of samples until error detection. The false alarm rate is reset to a plurality of different values such as 0.001%, 0.01%, 1%, 3%, and 5%, and the above process is repeated, thereby determining a parameter value combination satisfying a minimum average number of samples until error detection.

Table 4 Parameter values of alarm parameters

| N | M |
|---|---|
| 1 | 3 |
| 3 | 5 |
| 7 | 10 |
| 14 | 20 |
| ... | ... |

In some other embodiments, if the test set includes data with multiple occurrences where the instrument is not under control in reality, then upper and lower control limits and an average number of samples until error detection at the false alarm rate may be determined for each parameter value combination by using the test set, by way of: calculating monitoring indicators for said parameter value combination in the quality control model with said parameter value combination by using the actual test results for the target item and the patient information of the patient samples in the training set, and acquiring the upper and lower control limits based on the monitoring indicators for said parameter value combination and based on the false alarm rate, and inputting the actual test results for the target item of the patient samples in the test set together with the corresponding patient information into the quality control model with said parameter value combination, so as to obtain monitoring indicators corresponding to the test set; and comparing these monitoring indicators with the upper and lower control limits, so as to calculate an average of a number of samples required for each occurrence from when the instrument begins to be not under control in reality to when it is actually detected that the instrument is not under control by the quality control model, this is, to calculate the average number of samples until error detection ANPed.

In still other embodiments, another test set may be obtained, wherein the other test set includes actual test results for a target item and patient information of a plurality of patient samples. The actual test results are obtained by the testing instrument testing the patient samples, and patient information includes at least one of patient's disease, patient's age, patient's gender, or department to which the respective sample belongs. The other test set includes data with multiple occurrences where the instrument is not under control in reality. Here, upper and lower control limits and an average number of samples until error detection at the false alarm rate may be determined for each parameter value combination by using the other test set, by way of: calculating monitoring indicators for said parameter value combination in the quality control model with said parameter value combination by using the actual test results for the target item and the patient information of the patient samples in the training set, and acquiring the upper and lower control limits based on the monitoring indicators for said parameter value combination and based on the false alarm rate, and inputting the actual test results for the target item of the patient samples in the other test set together with the corresponding patient information into the quality control model with said parameter value combination, so as to obtain monitoring indicators corresponding to the other test set; and comparing these monitoring indicators with the upper and lower control limits, so as to calculate an average of a number of samples required for each occurrence from when the instrument begins to be not under control in reality to when it is actually detected that the instrument is not under control by the quality control model, this is, to calculate the average number of samples until error detection ANPed.

In some other embodiments not shown, M and N may also be parameters to be optimized of the quality control model, and parameters of the computational model, M, N, and optionally parameters of the truncation processing are optimized by:

setting a plurality of control limit positions for the quality control model, a plurality of SPC parameter values for the computational model, a plurality of alarm parameter value combinations M and N, and optionally a plurality of truncation ratios for the truncation processing;
determining a false alarm rate and an average number of sample until error detection for each parameter value combination of each control limit position, each SPC parameter value, each alarm parameter value combination, and optionally each truncation ratio, by:

calculating monitoring indicators for said parameter value combination in the quality control model with said parameter value combination by using the actual test results and the patient information in the training set, and calculating the false alarm rate of the model based on the upper and lower control limits in said parameter value combination and the monitoring indicators for said parameter value combination, and
calculating monitoring indicators for said parameter value combination with occurrence of an instrument out-of-control event in the quality control model with said parameter value combination by using a simulated data set including multiple occurrences where the instrument is simulated to be not under control or a real data set including multiple occurrences where the instrument is not under control in reality, and comparing the calculated monitoring indicators with the upper and lower control limits, so as to calculate an average of a number of samples required for each occurrence from when the instrument is simulated to begin to be not under control or the instrument begins to be not under control in reality to when it is actually detected that the instrument is not under control by the quality control model, as the average number of samples until error detection,

wherein the simulated data set is obtained by repeatedly adding deviations to the actual test results in the training set at different time points, and the real data set is obtained from the data of the plurality of patient samples or obtained by acquiring actual test results for the target item and the patient information of a plurality of additional patient samples; and

determining an optimal parameter value combination based on the false alarm rate of the model and the average number of samples until error detection for each parameter value combination, so as to construct an optimized quality control model.

In some embodiments, a parameter value combination with a minimum average number of samples until error detection among the parameter value combinations with a false alarm rate of the model lower than a preset false alarm rate may be selected as a final optimized parameter value combination for the quality control model.

In other embodiments, the false alarm rate of the model and the average number of samples until error detection for each parameter value combination may be weighted and then summed, and then a parameter value combination corresponding to a minimum sum is selected.

Taking that the statistical process control SPC algorithm is a moving average method as an example, the parameter of the moving average method-based computational model is a sliding window. parameters to be optimized of the quality control model include a sliding window, a truncation ratio, and an M&N alarm parameter combination. Upper and lower control limit positions (located at the upper and lower S/2 percentiles of the monitoring indicators respectively, where S is set to 0.001%, 0.01%, 0.1%, 1%, 3%, 5%) are set. The parameter value of the sliding window is set to 10, 20, 50, 70, and 100. The parameter value of the truncation ratio for the truncation processing is set to $\pm0\%$, $\pm1\%$, $\pm2\%$, and $\pm5\%$. The parameter value of the alarm parameter combination is set according to Table 4. Monitoring indicators for different parameter value combinations of the upper and lower control limits, the sliding window, the truncation ratio, and the alarm parameter combination are calculated by using the training set, and then a false alarm rate of the model is calculated based on the monitoring indicators and the upper and lower control limits. Subsequently, deviations are added randomly at a plurality of positions in the training set, a number of samples until error detection for each deviation is calculated, and then the calculated values are averaged out to obtain an average number of samples until error detection. A parameter value combination with a minimum average number of samples until error detection among the parameter value combinations with a false alarm rate of the model lower than a preset false alarm rate is selected as a final optimized parameter value combination for the quality control model.

In still other embodiments not shown, M and N may also be parameters to be optimized of the quality control model. Control limits of the quality control model, parameters of the computational model, M, N, and optionally parameters of the truncation processing are optimized by:

setting a preset false alarm rate of the quality control model, a plurality of control limit positions, a plurality of SPC parameter values for the computational model, a plurality of alarm parameter value combinations M and N, and optionally a plurality of truncation ratios for the truncation processing;

determining a false alarm rate for each parameter value combination of each control limit position, each SPC parameter value, each alarm parameter value combination, and optionally each truncation ratio, by: calculating monitoring indicators for said parameter value combination in the quality control model with said parameter value combination by using the actual test results and the patient information in the training set, and calculating the false alarm rate of the model based on the control limit positions in said parameter value combination and the monitoring indicators for said parameter value combination; and

selecting model parameter value combinations with a false alarm rate of the model lower than the preset false alarm rate, as candidate parameter value combinations;

determining an average number of samples until error detection for each candidate parameter value combination by: calculating monitoring indicators for said candidate parameter value combination with occurrence of an instrument out-of-control event in the quality control model with said candidate parameter value combination by using a simulated data set including multiple occurrences where the instrument is simulated to be not under control or a real data set including multiple occurrences where the instrument is not under control in reality, and comparing the calculated monitoring indicators with the upper and lower control limits in said candidate parameter value combination, so as to calculate an average of a number of samples required for each occurrence from when the instrument is simulated to begin to be not under control or the instrument begins to be not under control in reality to when it is actually detected that the instrument is not under control by the quality control model, as the average number of samples until error detection, wherein the simulated data set is obtained by repeatedly adding deviations to the actual test results in the training set at different time points, and the real data set is obtained from the data of the plurality of patient samples or obtained by acquiring actual test results for the target item and the patient information of a plurality of additional patient samples; and

using a candidate parameter value combination with a minimum average number of samples until error detection to construct an optimized quality control model.

Here, it should be understood that values of other parameters of the quality control model are fixed, for example, selected empirically, during optimization of the control limits of the quality control model, the parameters of the computational model, M, N, and optionally the parameters of the truncation processing. For example, the truncation ratio for the truncation processing may be fixed during optimization of the control limits of the quality control model, the parameters of the computational model, M, and N.

In some embodiments, in step S340, the quality control model may be verified as follows: the data of the test set is inputted into the quality control model constructed in step S330, it is determined whether the corresponding monitoring indicators fall within the upper and lower control limits, and if the monitoring indicators go beyond the upper or lower control limit, it indicates that a non-random deviation occurs in the system, and that the testing instrument is out of control, and therefore, an out-of-control alarm is generated. If the monitoring indicators are not beyond the upper and lower control limits, it indicates that the system is normal and the testing instrument is under control. If the test set includes data with multiple occurrences where the instrument is not under control in reality, the data may be used directly to verify the validity of the algorithm. In the case where no data with multiple occurrences where the instrument is not under control in reality exists in the test set, data with multiple occurrences where the instrument is simulated to be not under control is obtained by adding allowable deviations in the test set from a specified time point, so as to verify the detection performance of the algorithm model. By repeated simulation (adding deviations to the test set at different time points), all simulated numbers of samples until error detection can be averaged out to obtain an average number of samples until error detection, and the average number of samples until error detection is used to verify the performance of the quality control model.

In some embodiments, in step S320, quantifying the patient information of the patient samples in the training set, may include: respectively quantifying at least one of the patient information of the patient samples in the training set, especially at least one of patient's disease, patient's gender, or department to which the respective sample belongs, as a matrix.

Further, respectively quantifying the at least one of the patient information of the patient samples in the training set as the matrix, may include: quantifying each of the at least one of the patient samples in the training set as a one-dimensional matrix with a plurality of elements, wherein each element of the one-dimensional matrix represents one category, and said each of the patient information belongs to at least one of the categories represented by the elements.

Preferably, respectively quantifying the at least one of the patient information of the patient samples in the training set as a one-dimensional matrix with a plurality of elements, may include:

quantifying each of the at least one of the patient information of the patient samples in the training set as a one-dimensional matrix with a plurality of elements that is composed of 0s and 1s, wherein a value of the element representing the category to which said each of the patient information belongs is 1, and values of remaining elements are 0.

Alternatively or additionally, quantifying the patient information of the patient samples in the training set may include: quantifying the patient information of the patient samples in the training set as a fixed value, preferably as a fixed integer.

Alternatively or additionally, the patient information of the patient samples in the training set may be quantified through a lookup table method.

For specific quantification examples, reference may be made to the above detailed description of various embodiments of the method 100, which will not be repeated herein.

In some embodiments, the patient information includes patient' disease, patient' age, patient' gender, and department to which the respective sample belongs.

The various embodiments of the method 100 and the computer system 300 provided in the embodiments of the disclosure, and the advantages thereof, may correspondingly apply to the model construction method 300 provided in the embodiments of the disclosure, and thus will not be repeated.

An embodiment of the disclosure further relates to an application of the quality control model constructed according to the above-described method 300 in quality control of a testing instrument, wherein actual test results for the target item and patient information of a plurality of patient samples are acquired, wherein the actual test results are obtained by the testing instrument performing tests on the patient samples, and the patient information includes at least one of patient's disease, patient's age, patient's gender, or department to which the respective sample belongs; the patient information of the plurality of patient samples is quantified and the quantified patient information is inputted together with the actual test results into the quality control model, to acquire monitoring indicators; and whether or not the testing instrument is under control is determined based on the monitoring indicators.

A person skilled in the art should understand that the embodiments of the disclosure may be provided as a method, a system, or a computer program product. Therefore, the embodiments of the disclosure may be implemented in the form of a hardware embodiment, a software embodiment, or a combination thereof. Moreover, the embodiments of the disclosure may be implemented in the form of a computer program product that is implemented on one or more computer-usable storage media (including a disk memory, an optical memory, and the like) that include computer-usable program codes.

The embodiments of the disclosure are described with reference to flowcharts and/or block diagrams of the methods, devices (systems), and computer program products according to the embodiments of the disclosure. It should be understood that each procedure and/or block in the flowcharts and/or block diagrams, and combinations of the procedures and/or blocks in the flowcharts and/or block diagrams may be implemented by computer program operations. These computer program operations may be provided to a processor of a general-purpose computer, a special-purpose computer, an embedded processor or another programmable data processing device to create a machine, such that the operations executed by the processor of the computer or the other programmable data processing device create a device for implementing functions specified in one or more procedures in the flowcharts and/or one or more blocks in the block diagrams.

These computer program operations may also be stored in a computer-readable storage medium that may direct a computer or other programmable data processing device to operate in a specific manner, such that the operations stored in the computer-readable storage medium create a manufacture article including an operation device, and the operation device implements the functions specified in one or more procedures in the flowcharts and/or one or more blocks in the block diagrams.

These computer program operations may also be loaded onto a computer or other programmable data processing devices to enable a series of operation steps to be executed on the computer or other programmable devices to perform computerimplemented processing, such that the operations executed on the computer or other programmable devices provide steps for implementing the functions specified in one or more procedures in the flowcharts and/or one or more blocks in the block diagrams.

The features or combinations thereof mentioned above in the description, accompanying drawings, and claims can be combined with each other arbitrarily or used separately as long as they are meaningful within the scope of the disclosure and do not contradict each other. The advantages and features described with respect to the method provided herein are correspondingly applicable to the system and application provided herein, and vice versa.

The foregoing description merely relates to the preferred embodiments of the disclosure, and is not intended to limit the patent scope of the disclosure. All equivalent variations made by using the content of the specification and the accompanying drawings of the disclosure from the concept of the disclosure, or the direct/indirect applications of the contents in other related technical fields all fall within the scope of patent protection of the disclosure.

**Claims**

1. A method for patient-based real-time quality control (PBRTQC), comprising:

   while a testing instrument performs real-time tests on current patient samples for a target item, acquiring actual test results of the current patient samples obtained by the testing instrument for the target item and patient information of the current patient samples, wherein the patient information comprises at least one of patient's disease, patient's age, patient's gender or ordering department for the target item;
   quantifying the patient information of the current patient samples and inputting the quantified patient information into a pre-constructed machine learning model to obtain output results of the machine learning model as predicted test results for the target item, wherein the machine learning model is trained by using actual test results for the target item and patient information of a plurality of historical patient samples;
   acquiring monitoring indicators based on the actual test results and the predicted test results of the current patient samples; and
   determining whether or not the testing instrument is under control based on the monitoring indicators, and, if it is determined that the testing instrument is not under control, outputting an alarm prompt indicating as such.

2. The method of claim 1, wherein acquiring monitoring indicators based on the actual test results and the predicted test results of the current patient samples, comprises:

   calculating differences between the actual test results and the predicted test results; and
   inputting the differences into a statistical process control (SPC) algorithm-based computational model, to obtain output results of the computational model as the monitoring indicators.

3. The method of claim 1 or 2, wherein quantifying the patient information of the current patient samples, comprises: respectively quantifying at least one of the patient information of the current patient samples, especially at least one of patient's disease, patient's gender or ordering department for the target item, as a matrix.

4. The method of claim 3, wherein respectively quantifying at least one of the patient information of the current patient samples as a matrix, comprises:

quantifying each of the at least one of the patient information of the current patient samples as a one-dimensional matrix with a plurality of elements, wherein each element of the one-dimensional matrix represents one category, and said each of the patient information belongs to at least one of the categories represented by the elements; and preferably, quantifying each of the at least one of the patient information of the current patient samples as a one-dimensional matrix with a plurality of elements, comprises:
quantifying each of the at least one of the patient information of the current patient samples as a one-dimensional matrix with a plurality of elements that is composed of 0s and 1s, wherein a value of the element representing the category to which said each of the patient information belongs is 1, and values of remaining elements are 0.

5. The method of claim 1 or 2, wherein quantifying the patient information of the current patient samples, comprises: respectively quantifying at least one of the patient information of the current patient samples, especially patient's age, as a fixed value, preferably as a fixed integer.

6. The method of any one of claims 1 to 5, wherein at least one of the patient information of the current patient samples is quantified through a lookup table method.

7. The method of any one of claims 1 to 6, wherein the patient information comprises patient's disease, patient's age, patient's gender and ordering department for the target item.

8. The method of any one of claims 1 to 7, wherein acquiring monitoring indicators based on the actual test results and the predicted test results of the current patient samples, comprises:

performing data processing on the actual test results, wherein the data processing comprises truncation processing and/or normalization; and
acquiring the monitoring indicators based on the actual test results that are subjected to the data processing and the predicted test results.

9. The method of any one of claims 1 to 8, wherein the machine learning model is a neural network model.

10. The method of any one of claims 1 to 8, wherein the machine learning model is a support vector machine (SVM)- and/or linear discriminant analysis (LDA)-based machine learning model.

11. The method of any one of claims 1 to 10, wherein determining whether or not the testing instrument is under control based on the monitoring indicators, comprises: successively acquiring monitoring indicators of a consecutive first number of current patient samples in chronological order, and determining whether or not the testing instrument is under control based on the monitoring indicators of the consecutive first number of current patient samples, wherein when, among the monitoring indicators of the consecutive first number of current patient samples, there at least exist monitoring indicators of a second number of current patient samples that exceed a control limit, an alarm prompt is outputted that indicates the testing instrument is not under control, wherein the first number is greater than the second number, and the second number is greater than 1.

12. The method of claim 2, wherein parameters of the computational model are optimized by:

acquiring actual test results for the target item and patient information of a plurality of historical patient samples, wherein the actual test results are obtained by the testing instrument performing tests on the plurality of historical patient samples in a controlled state;
setting a false alarm rate;
setting SPC parameter values for the computational model;
determining, at the false alarm rate, an upper control limit and a lower control limit and an average number of samples until error detection, for each SPC parameter value, by:

calculating monitoring indicators for said SPC parameter value in the computational model with said SPC parameter value by using the actual test results and the patient information of the plurality of historical patient samples, and acquiring the upper and lower control limits based on the monitoring indicators for said SPC parameter value and based on the false alarm rate, and

calculating monitoring indicators for said SPC parameter value with an occurrence of an instrument out-of-control event in the computational model with said SPC parameter value by using a simulated data set including multiple occurrences where the instrument is simulated to be not under control or a real data set including multiple occurrences where the instrument is not under control in reality, and comparing the calculated monitoring indicators with the upper and lower control limits, so as to calculate an average of a number of samples required for each occurrence from when the instrument is simulated to begin to be not under control or the instrument begins to be not under control in reality to when it is actually detected that the instrument is not under control, as the average number of samples until error detection,

wherein the simulated data set is obtained by repeatedly adding deviations to the actual test results of the plurality of historical patient samples at different time points, and the real data set is obtained from the actual test results and the patient information of the plurality of historical patient samples or obtained by acquiring actual test results for the target item and patient information of a plurality of additional historical patient samples; and

selecting a SPC parameter value with a minimum average number of samples until error detection and selecting upper and lower control limits corresponding to the selected SPC parameter value, to construct an optimized computational model.

13. The method of claim 8, wherein parameters of the truncation processing and parameters of the computational model are optimized by:

acquiring actual test results for the target item and patient information of a plurality of historical patient samples, wherein the actual test results are obtained by the testing instrument performing tests on the historical patient samples in a controlled state;
setting a false alarm rate;
setting truncation ratios for the truncation processing and SPC parameter values for the computational model;
determining, at the false alarm rate, an upper control limit and a lower control limit and an average number of samples until error detection for each parameter value combination of each truncation ratio and each SPC parameter value by:

calculating monitoring indicators for said parameter value combination in the computational model with said parameter value combination by using the actual test results and the patient information of the plurality of historical patient samples, and acquiring the upper and lower control limits based on the monitoring indicators for said parameter value combination and based on the false alarm rate, and

calculating monitoring indicators for said parameter value combination with occurrence of an instrument out-of-control event in the computational model with said parameter value combination by using a simulated data set including multiple occurrences where the instrument is simulated to be not under control or a real data set including multiple occurrences where the instrument is not under control in reality, and comparing the calculated monitoring indicators with the upper and lower control limits, so as to calculate an average of a number of samples required for each occurrence from when the instrument is simulated to begin to be not under control or the instrument begins to be not under control in reality to when it is actually detected that the instrument is not under control, as the average number of samples until error detection,

wherein the simulated data set is obtained repeatedly by adding deviations to the actual test results of the plurality of historical patient samples at different time points, and the real data set is obtained from the actual test results and the patient information of the plurality of historical patient samples or obtained by acquiring actual test results for the target item and patient information of a plurality of additional historical patient samples; and

selecting a parameter value combination with a minimum average number of samples until error detection and upper and lower control limits corresponding to the selected parameter value combination, so as to construct an optimized truncation processing and an optimized computational model.

14. A computer system for patient-based real-time quality control (PBRTQC), comprising:

   a data acquisition module, configured to, while a testing instrument performs real-time tests on current patient samples for a target item, acquire actual test results of the current patient samples obtained by the testing instrument for the target item and patient information of the current patient samples, wherein the patient information comprises at least one of patient's disease, patient's age, and patient's gender, or ordering department for the target item;
   a predicted result acquisition module, configured to quantify the patient information of the current patient samples and input the quantified patient information into a pre-constructed machine learning model to obtain output results of the machine learning model as predicted test results for the target item, wherein the machine learning model is trained by using actual test results for the target item and patient information of a plurality of historical patient samples;
   a monitoring indicator acquisition module, configured to acquire monitoring indicators based on the actual test results and the predicted test results of the current patient samples; and
   a quality control module, configured to determine whether or not the testing instrument is under control based on the monitoring indicators, and, if it is determined that the testing instrument is not under control, output an alarm prompt indicating as such.

15. The computer system of claim 14, wherein the monitoring indicator acquisition module is further configured to:

   calculate differences between the actual test results and the predicted test results; and
   input the differences into a statistical process control (SPC) algorithm-based computational model, to obtain output results of the computational model as the monitoring indicators.

16. The computer system of claim 14 or 15, wherein the predicted result acquisition module is further configured to respectively quantify at least one of the patient information of the current patient samples, especially at least one of patient's disease, patient's gender, or ordering department for the target item, as a matrix.

17. The computer system of claim 16, wherein the predicted result acquisition module is further configured to quantify each of the at least one of the patient information of the current patient samples as a one-dimensional matrix with a plurality of elements, wherein each element of the one-dimensional matrix represents one category, and said each of the patient information belongs to at least one of categories represented by the elements; and
   preferably, the predicted result acquisition module is further configured to quantify each of the at least one of the patient information of the current patient samples as a one-dimensional matrix with a plurality of elements that is composed of 0s and 1s, wherein a value of the element representing the category to which said each of the patient information belongs is 1, and values of remaining elements are 0.

18. The computer system of claim 14 or 15, wherein the predicted result acquisition module is further configured to quantify the patient information of the current patient samples as a fixed value, preferably as a fixed integer.

19. The computer system of any one of claims 14 to 18, wherein the predicted result acquisition module is further configured to quantify the patient information of the current patient samples through a lookup table method.

20. The computer system of any one of claims 14 to 19, wherein the patient information comprises patient's disease, patient's age, patient's gender, and ordering department for the target item.

21. The computer system of any one of claims 14 to 10, wherein the monitoring indicator acquisition module is further configured to:

   perform data processing on the actual test results, wherein the data processing comprises truncation processing and/or normalization; and
   acquire the monitoring indicators based on the actual test results that are subjected to the data processing and the predicted test results.

22. The computer system of any one of claims 14 to 21, wherein the machine learning model is a neural network model; or the machine learning model is a support vector machine (SVM)- and/or linear discriminant analysis (LDA)-based machine learning model.

23. The computer system of any one of claims 14 to 22, wherein the quality control module is further configured to successively acquire monitoring indicators of a consecutive first number of current patient samples in chronological order, and determine whether or not the testing instrument is under control based on the monitoring indicators of the consecutive first number of current patient samples; and when, among the monitoring indicators of the consecutive first number of current patient samples, there at least exist monitoring indicators of a second number of current patient samples that exceed a control limit, output an alarm prompt that indicates the testing instrument is not under control, wherein the first number is greater than the second number, and the second number is greater than 1.

24. The computer system of claim 15, wherein the monitoring indicator acquisition module is optimized by:

acquiring actual test results for the target item and patient information of a plurality of historical patient samples, wherein the actual test results are obtained by the testing instrument performing tests on the plurality of historical patient samples in a controlled state;
setting a false alarm rate;
setting SPC parameter values for the computational model;
determining, at the false alarm rate, an upper control limit and a lower control limit and an average number of samples until error detection, for each SPC parameter value by:

calculating monitoring indicators for said SPC parameter value in the computational model with said SPC parameter value by using the actual test results and the patient information of the plurality of historical patient samples, and acquiring the upper and lower control limits based on the monitoring indicators for said SPC parameter value and based on the false alarm rate, and
calculating monitoring indicators for said SPC parameter value with occurrence of an instrument out-of-control event in the computational model with said SPC parameter value by using a simulated data set including multiple occurrences where the instrument is simulated to be not under control or a real data set including multiple occurrences where the instrument is not under control in reality, and comparing the calculated monitoring indicators with the upper and lower control limits, so as to calculate an average of a number of samples required for each occurrence from when the instrument is simulated to begin to be not under control or the instrument begins to be not under control in reality to when it is actually detected that the instrument is not under control, as the average number of samples until error detection,
wherein the simulated data set is obtained by repeatedly adding deviations to the actual test results of the plurality of historical patient samples at different time points, and the real data set is obtained from the actual test results and the patient information of the plurality of historical patient samples or obtained by acquiring actual test results for the target item and patient information of a plurality of additional historical patient samples; and

selecting an SPC parameter value with a minimum average number of samples until error detection and upper and lower control limits corresponding to the selected SPC parameter value, to construct an optimized computational model.

25. The computer system of claim 21, wherein the monitoring indicator acquisition module is optimized by:

acquiring actual test results for the target item and patient information of a plurality of historical patient samples, wherein the actual test results are obtained by the testing instrument performing tests on the historical patient samples in a controlled state;
setting a false alarm rate;
setting truncation ratios for the truncation and SPC parameter values for the computational model;
determining, at the false alarm rate, an upper control limit and a lower control limit and an average number of samples until error detection for each parameter value combination of each truncation ratio and each SPC parameter value by:

calculating monitoring indicators for said parameter value combination in the computational model with said parameter value combination by using the actual test results and the patient information of the plurality of historical patient samples, and acquiring the upper and lower control limits based on the monitoring indicators for said parameter value combination and based on the false alarm rate, and
calculating monitoring indicators for said parameter value combination with occurrence of an instrument out-of-control event in the computational model with said parameter value combination by using a simulated data set including multiple occurrences where the instrument is simulated to be not under control or a real data set including multiple occurrences where the instrument is not under control in reality, and comparing the calculated monitoring indicators with the upper and lower control limits, so as to calculate an average of a number of samples required for each occurrence from when the instrument is simulated to begin to be not under control or the instrument begins to be not under control in reality to when it is actually detected that the instrument is not under control, as the average number of samples until error detection,

wherein the simulated data set is obtained by repeatedly adding deviations to the actual test results of the plurality of historical patient samples at different time points, and the real data set is obtained from the actual test results and the patient information of the plurality of historical patient samples or obtained by acquiring actual test results for the target item and patient information of a plurality of additional historical patient samples; and

selecting a parameter value combination with a minimum average number of samples until error detection and upper and lower control limits corresponding to the selected parameter value combination, so as to construct an optimized truncation processing and an optimized computational model.

26. A method for constructing a quality control model for patient-based real-time quality control (PBRTQC), comprising:

acquiring data of a plurality of patient samples and dividing the data into a training set and a test set, the data comprising actual test results for a target item and patient information of the plurality of patient samples, wherein the actual test results are obtained by a testing instrument performing tests on the patient samples, and the patient information comprises at least one of patient's disease, patient's age, patient's gender, or ordering department for the target item, wherein the data in the training set is obtained when the testing instrument is in a controlled state; building a machine learning model using the training set by way of quantifying the patient information of the patient samples in the training set, and building a machine learning model between the quantified patient information and the actual test results in the training set, wherein the machine learning model satisfies the following formula:

$$X_t = f(x_{1t}, x_{2t}, \cdots, x_{nt}) + \varepsilon_t$$

where $X_t$ is the actual test results in the training set, $f(x_{1t}, x_{2t}, \cdots, x_{nt})$ is the machine learning model, $x_{1t}, x_{2t}, \cdots, x_{nt}$ represents the quantified patient information, and $\varepsilon_t$ represents a residual of estimation of $X_t$ using the machine learning model; constructing, based on the machine learning model, a quality control model for patient-based real-time quality control; and verifying the quality control model by using the test set.

27. The method of claim 26, wherein the machine learning model is a neural network model; or the machine learning model is a support vector machine (SVM)- and/or linear discriminant analysis (LDA)-based machine learning model.

28. The method of claim 26 or 27, wherein constructing, based on the machine learning model, the quality control model for patient-based real-time quality control, comprises: constructing the quality control model based on the machine learning model and a statistical process control (SPC) algorithm-based computational model, wherein the residual $\varepsilon_t$ is inputted into the SPC algorithm-based computational model, to obtain an output of the computational model as a monitoring indicator of the quality control model.

29. The method of claim 28, wherein parameters of the computational model are optimized by:

setting a false alarm rate; setting SPC parameter values for the computational model; determining, at the false alarm rate, an upper control limit and lower control limit and an average number of samples until error detection for each SPC parameter value, by:

calculating monitoring indicators for said SPC parameter value in the quality control model with said SPC parameter value by using the actual test results and the patient information in the training set, and acquiring the upper and lower control limits based on the monitoring indicators for said SPC parameter value and based on the false alarm rate, and calculating monitoring indicators for said SPC parameter value with occurrence of an instrument out-of-control event in the quality control model with said SPC parameter value by using a simulated data set including multiple occurrences where the instrument is simulated to be not under control or a real data set including multiple occurrences where the instrument is not under control in reality, and comparing the calculated monitoring indicators with the upper and lower control limits, so as to calculate an average of a number of samples required for each occurrence from when the instrument is simulated to begin to be not under control or the instrument begins to be not under control in reality to when it is actually detected that the instrument is not under control by the quality control model, as the average number of samples until error detection,

wherein the simulated data set is obtained by repeatedly adding deviations to the actual test results in the training set at different time points, and the real data set is obtained from the data of the plurality of patient samples or obtained by acquiring actual test results for the target item and patient information of a plurality of additional patient samples; and

selecting an SPC parameter value with a minimum average number of samples until error detection and upper and lower control limits corresponding to the selected SPC parameter value, to construct an optimized quality control model.

30. The method of claim 28, wherein data processing is performed on the actual test results for the target item of the patient samples in the training set before building the machine learning model, wherein the data processing comprises truncation processing and/or normalization.

31. The method of claim 30, wherein parameters of the truncation processing and parameters of the computational model are optimized by:

setting a false alarm rate;
setting truncation ratios for the truncation and SPC parameter values for the computational model;
determining, at the false alarm rate, an upper control limit and a lower control limit and an average number of samples until error detection for each parameter value combination of each truncation ratio and each SPC parameter value by:

calculating monitoring indicators for said parameter value combination in the quality control model with said parameter value combination by using the actual test results and the patient information in the training set, and acquiring the upper and lower control limits based on the monitoring indicators for said parameter value combination and based on the false alarm rate, and

calculating monitoring indicators for said parameter value combination with occurrence of an instrument out-of-control event in the quality control model with said parameter value combination by using a simulated data set including multiple occurrences where the instrument is simulated to be not under control or a real data set including multiple occurrences where the instrument is not under control in reality, and comparing the calculated monitoring indicators with the upper and lower control limits, so as to calculate an average of a number of samples required for each occurrence from when the instrument is simulated to begin to be not under control or the instrument begins to be not under control in reality to when it is actually detected that the instrument is not under control by the quality control model, as the average number of samples until error detection,

wherein the simulated data set is obtained by repeatedly adding deviations to the actual test results in the training set at different time points, and the real data set is obtained from the data of the plurality of patient samples or obtained by acquiring actual test results for the target item and patient information of a plurality of additional patient samples; and

selecting a parameter value combination with a minimum average number of samples until error detection and upper and lower control limits corresponding to the selected parameter value combination, so as to construct an optimized quality control model.

32. The method of any one of claims 26 to 31, wherein quantifying the patient information of the patient samples in the training set, comprises:
respectively quantifying at least one of the patient information of the patient samples in the training set, especially at least one of patient's disease, patient's gender, or ordering department for the target item, as a matrix.

33. The method of claim 32, wherein respectively quantifying at least one of the patient information of the patient samples in the training set as the matrix, comprises:

quantifying each of the at least one of the patient samples in the training set as a one-dimensional matrix with a plurality of elements, wherein each element of the one-dimensional matrix represents one category, and said each of the patient information belongs to at least one of the categories represented by the elements; and preferably, quantifying each of the at least one of the patient samples in the training set as a one-dimensional matrix with a plurality of elements, comprises:

quantifying each of the at least one of the patient samples in the training set as a one-dimensional matrix with a plurality of elements that is composed of 0s and 1s, wherein a value of the element representing the category to which said each of the patient information belongs is 1, and values of remaining elements are 0.

34. The method of any one of claims 26 to 31, wherein quantifying the patient information of the patient samples in the training set, comprises:
quantifying the patient information of the patient samples in the training set as a fixed value, preferably as a fixed integer.

35. The method of any one of claims 26 to 34, wherein the patient information of the patient samples in the training set is quantified through a lookup table method.

36. The method of any one of claims 26 to 35, wherein the patient information comprises patient's disease, patient's age, patient's gender, and ordering department for the target item.

37. A method for patient-based real-time quality control, comprising:

while a testing instrument performs real-time tests on current patient samples for a target item, acquiring actual test results of the current patient samples obtained by the testing instrument for the target item and patient information of the current patient samples, wherein the patient information comprises at least one of patient's disease, patient's age, patient's gender, or ordering department for the target item;
quantifying the patient information of the current patient samples and inputting the quantified patient information into a pre-constructed machine learning model to obtain output results of the machine learning model as predicted test results for the target item, wherein the machine learning model is obtained by using actual test results for the target item and patient information of a plurality of historical patient samples;
acquiring monitoring indicators based on the actual test results and the predicted test results of the current patient sample; and
determining whether or not the testing instrument is under control, based on the monitoring indicators, and/or determining whether or not to give an alarm prompt for indicating that the testing instrument is not under control, based on the monitoring indicators.

<u>100</u>

S110

acquiring actual test results for a target item and patient information of patient samples

S120

quantifying the patient information and inputting the quantified patient information into a machine learning model to obtain output results of the machine learning model as predicted test results for the target item

S130

acquiring monitoring indicators based on the actual test results and the predicted test results

S140

determining whether or not the testing instrument is under control, based on the monitoring indicators, and/or determining whether or not to give an alarm prompt indicating that the testing instrument is out of control, based on the monitoring indicators

# Fig. 1

Fig. 2

**Fig. 3**

<u>200</u>

210

220

230

240

# Fig. 4

300

S310 — acquiring data of a plurality of patient samples and dividing the data into a training set and a test set

S320 — building a machine learning model by using the training set

S330 — constructing a quality control model based on the machine learning model

S340 — verifying the quality control model by using the test set

Fig. 5

n feature
nodes

k nodes

Output 1
node

Fig. 6

<u>330</u>

S331a

setting a false alarm rate

S332a

setting SPC parameter values for the computational model

S333a

determining, at the false alarm rate, upper and lower control limits and an average number of samples until error detection for each SPC parameter value

S334a

selecting an SPC parameter value with a minimum average number of Samples until error detection and upper and lower control limits corresponding to the selected SPC parameter value

## Fig. 7

330

S331b

setting a false alarm rate

S332b

setting truncation ratios for the truncation processing and SPC parameter values for the computational model

S333b

determining, at the false alarm rate, upper and lower control limits and an average number of samples until error detection for each parameter value combination

S334b

selecting a parameter value combination with a minimum average number of samples until error detection and upper and lower control limits corresponding to the selected parameter value combination

# Fig. 8

330

S331c

setting a false alarm rate

S332c

setting truncation ratios for the truncation processing, parameter values for M, parameter values for N, and SPC parameter values for the computational model

S333c

determining at the false alarm rate, upper and lower control limits and an average number of samples until error detection for each parameter value combination

S334c

selecting a parameter value combination with a minimum average number of samples until error detection and upper and lower control limits corresponding to the selected parameter value combination

# Fig. 9

## INTERNATIONAL SEARCH REPORT

| International application No. |
| --- |
| **PCT/CN2024/095713** |

| A. | CLASSIFICATION OF SUBJECT MATTER |
| --- | --- |

G16H40/20(2018.01)i

According to International Patent Classification (IPC) or to both national classification and IPC

| B. | FIELDS SEARCHED |
| --- | --- |

Minimum documentation searched (classification system followed by classification symbols)

IPC: G16H

Documentation searched other than minimum documentation to the extent that such documents are included in the fields searched

Electronic data base consulted during the international search (name of data base and, where practicable, search terms used)

CNABS; CNTXT; CNKI; WPABS; DWPI; USTXT; WOTXT; EPTXT: 患者, 信息, 样本, 数据, 质量, 控制, 检测仪, 分析仪, 医疗设备, 报警, 校正, 校准, 监控, 机器学习, 神经网络, 参数, 模型, 优化, SPC, patient data, quality, control, instrument, medical equipment, monitor, warning, machine learning, neural network, model optimization

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | CN 115910364 A (BEIJING CHAO-YANG HOSPITAL, CAPITAL MEDICAL UNIVERSITY et al.) 04 April 2023 (2023-04-04) description, paragraphs [0081]-[0291] | 1-11, 14-23, 26-28, 30, 32-37 |
| Y | CN 115910364 A (BEIJING CHAO-YANG HOSPITAL, CAPITAL MEDICAL UNIVERSITY et al.) 04 April 2023 (2023-04-04) description, paragraphs [0081]-[0291] | 12, 13, 24, 25, 29, 31 |
| Y | CN 114330859 A (ZHONGSHAN HOSPITAL, FUDAN UNIVESITY) 12 April 2022 (2022-04-12) description, paragraphs [0039]-[0089] | 12, 13, 24, 25, 29, 31 |
| A | CN 113559423 A (WEST CHINA HOSPITAL, SICHUAN UNIVERSITY) 29 October 2021 (2021-10-29) description, paragraphs [0030]-[0043] | 1-37 |
| A | CN 115691722 A (BEIJING CHAO-YANG HOSPITAL, CAPITAL MEDICAL UNIVERSITY) 03 February 2023 (2023-02-03) description, paragraphs [0075]-[0276] | 1-37 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **17 July 2024** | **14 August 2024** |

| Name and mailing address of the ISA/CN | Authorized officer |
| --- | --- |
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2024/095713**

| C. | DOCUMENTS CONSIDERED TO BE RELEVANT | |
|---|---|---|
| Category* | Citation of document, with indication, where appropriate, of the relevant passages | Relevant to claim No. |
| A | CN 107704986 A (INNER MONGOLIA MENGNIU DAIRY (GROUP) CO., LTD.) 16 February 2018 (2018-02-16)<br>description, paragraphs [0027]-[0143] | 1-37 |

Form PCT/ISA/210 (second sheet) (July 2022)

**INTERNATIONAL SEARCH REPORT**
Information on patent family members

International application No.

**PCT/CN2024/095713**

| Patent document cited in search report | | | Publication date (day/month/year) | Patent family member(s) | | | Publication date (day/month/year) |
|---|---|---|---|---|---|---|---|
| CN | 115910364 | A | 04 April 2023 | None | | | |
| CN | 114330859 | A | 12 April 2022 | None | | | |
| CN | 113559423 | A | 29 October 2021 | CN | 113559423 | B | 02 June 2023 |
| CN | 115691722 | A | 03 February 2023 | CN | 115691722 | B | 29 September 2023 |
| CN | 107704986 | A | 16 February 2018 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2023097857 W